# EUROPEAN PATENT APPLICATION

(11) **EP 4 397 660 A1**
(43) Date of publication of application: **10.07.2024**
(21) Application number: 22863470.5
(22) Date of filing: 30.08.2022
(51) Int. Cl.: C07D 403/12, C07D 403/14, C07D 413/12, C07D 413/14, A61K 31/501, A61P 35/00, A61P 19/02, A61P 17/00, A61P 37/02, A61P 29/00

(54) **COMPOUND AS TYK2/JAK1 PSEUDOKINASE DOMAIN INHIBITOR, AND SYNTHESIS AND USE METHODS**

(30) Priority: 31.08.2021 CN 202111013061
(71) Applicant: Zhejiang Wenda Pharmaceutical Technology Co., Ltd., Hangzhou, Zhejiang 310018 (CN)
(72) Inventor: WANG, Nenghui, Hangzhou, Zhejiang 310018 (CN)
(74) Representative: Lavoix
(86) International application number: PCT/CN2022/115930
(87) International publication number: WO 2023/030335

(57) **Abstract**

The present invention provides a compound as a TYK2/JAK1 pseudokinase domain inhibitor, and synthesis and use methods. Specifically, provided is a compound or a pharmaceutically acceptable salt thereof, the compound is as represented by formula A, and respective groups are as defined herein.

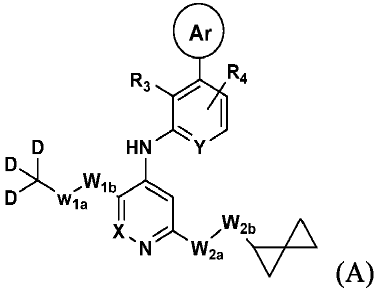

## Description

### TECHNICAL FIELD

The present invention belongs to the field of medicine, specifically, relates to a compound as TYK2 pseudokinase (Pseudokinase, JH2) domain inhibitor, and synthesis methods and use.

### BACKGROUND

Janus kinase (JAK) is an intracellular non receptor tyrosine kinase that mediates the signal transduction and activation of various cytokines. The JAK kinase family is divided into four subtypes: JAK1, 2, 3, and TYK2. Each subtype mediates different types of cytokine signaling pathways. JAK1, 2, and TYK2 are expressed in various tissues and cells in the human body, while JAK3 is mainly expressed in hematopoietic tissue cells.

TYK2 is the earliest subtype discovered in the JAK family, mediating the functions of cytokines such as IFN- a, IL-6, IL-10, IL-12, and IL-23. Studies have shown that TYK2 deletion mutations can effectively inhibit the occurrence of immune diseases such as allergies, autoimmune diseases, and inflammation. IL-23 plays a crucial role in the occurrence and development of psoriasis. Recent studies have shown that the pathogenesis of psoriasis is the secretion of IL-23 caused by the activation of antigen presenting cells APC by endogenous unknown antigen. IL-23 activates Thl7 cells to secrete cytokines such as IL-17, inducing keratinocyte differentiation and secretion of IL-23, further stimulating inflammation and keratinocyte proliferation to produce psoriasis. TYK2 and JAK2 jointly mediate the downstream signaling pathway of IL-23, Inhibition of JAK2 may lead to anemia and other blood related side effects. Therefore, inhibiting TYK2 while avoiding the inhibition of JAK2 is a good strategy for inhibition of IL-23 signaling pathway. The first oral Tofacitinib belongs to a non-selective JAK inhibitor, which has significant inhibitory activity on JAK1, 2, 3, and TYK2. It can targetedly inhibit the catalytic domain (JH1 or " homology 1 domain of JAK protein") that binds to the adenosine triphosphate (ATP) site, and prevent phosphorylation of downstream kinase catalytic activity and the resulting pathway signal transduction by blocking ATP. The inhibition of Tofacitinib on the activities of other subtypes such as JAK1, 2, 3, and TYK2 has increased its efficacy, but also brings serious side effects, including infections, tuberculosis, tumors, anemia, liver damage, and increased cholesterol. Due to the correlation between JAK2 activity and red blood cell differentiation and lipid metabolism processes, some of the above-mentioned side effects such as anemia are considered being related to the insufficient selectivity of Tofacitinib to JAK2, and are caused by the non-selective inhibition of the drug.

Given the excellent efficacy and the serious side effects associated with multiple targets of JAK non-selective inhibitors, there is an urgent need for an inhibitor for JAK kinase family in this field. Such inhibitor is functionally distinct from the binding catalytic domain (JH1) of previous JAK inhibitors, selectively binds to the pseudokinase (JH2) domain (such as JH2 of TYK2 and JAK1), inhibits the function of the pseudokinase (JH2) domain, avoids inhibition on other subtypes such as JAK1, 2, 3, TYK2 or kinase domain (JH1) thereof, and increases the safety of inhibitor for TYK2 and JAK1 pseudokinase (JH2). Thereby, the inhibitor can be used for and has enormous clinical potential in the treatment of various autoimmune and inflammatory related diseases, that is related to the TYK2 and JAK1 pseudokinase (JH2) domain function, including psoriasis, lupus erythematosus, inflammatory bowel disease, psoriatic arthritis, arthritis, dermatitis, lupus nephritis, neuroinflammation such as multiple sclerosis and senile dementia, ankylosing spondylitis, hidradenitis suppurativa, respiratory disease, diabetes, inflammatory eye disease, hepatitis, cardiovascular disease, systemic sclerosis, organ transplantation, alopecia areata, acne, eczema, vitiligo, sjogren's syndrome, viral inflammation, and some cancers.

Therefore, there is an urgent need in this field to develop TYK2 pseudokinase (JH2) domain inhibitors having novel structures with high safety, and excellent efficacy.

### SUMMARY OF THE INVENTION

The purpose of the present invention is to provide a class of TYK2 pseudokinase (JH2) domain inhibitor having novel structure with high safety, and excellent efficacy.

In the first aspect of the present invention, provided is a compound or a pharmaceutically acceptable salt thereof, wherein the compound is as shown in formula A, 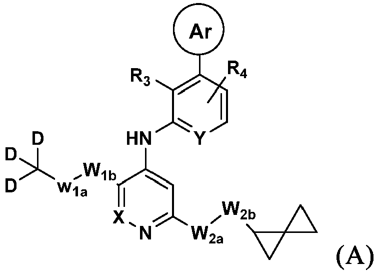 wherein,
Ring Ar is a five-membered heteroaromatic ring; and the five-membered heteroaromatic ring is optionally substituted with 1, 2, 3 or 4 substituents selected from the group consisting of: halogen, substituted or unsubstituted C₁₋₆ alkyl, substituted or unsubstituted C₁₋₆ hydroxyalkyl, substituted or unsubstituted C₁₋₄ alkylene-O-C₁₋₆ alkyl, substituted or unsubstituted C₁₋₄ alkylene-S-C₁₋₆ alkyl;
Y is CH or N;
X is CH or N;
R₃ is selected from the group consisting of H, halogen, substituted or unsubstituted C₁₋₆ alkyl, hydroxyl, substituted or unsubstituted C₁₋₆ alkoxy, substituted or unsubstituted C₁₋₆ alkylthiol;
R₄ is absent, or means 1 or 2 substituents selected from the group consisting of halogen, substituted or unsubstituted C₁₋₃alkyl;
W₁ₐ and W_{1b} are each independently selected from the group consisting of null (single bond), -O-, -S-, -SO₂₋, -CO-, -NR₅-; and at least one of W₁ₐ and W_{1b} is not null;
W₂ₐ and W_{2b} are each independently selected from the group consisting of null (single bond), -O-, -S-, -SO₂₋, -CO-, -NR₅-; and at least one of W₂ₐ and W_{2b} is not null;
R₅ is each independently selected from the group consisting of H, substituted or unsubstituted C₁₋₄alkyl;
unless otherwise specified, said substituted means that one or more (such as 1, 2, or 3) hydrogen in the group are substituted with substituents selected from the group consisting of halogen, C₁₋₄ alkyl, and C₁₋₄ haloalkyl.

In another preferred embodiment, the five-membered heteroaryl is a five-membered heteroaryl containing 1, 2 or 3 heteroatoms selected from O, S and N as ring atom; preferably, the five-membered heteroaryl is a five-membered heteroaryl containing 2 nitrogen heteroatoms and 0 or 1 heteroatom selected from O, S and N.

In another preferred embodiment, the five-membered heteroaryl is selected from the group consisting of:

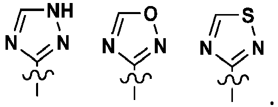

In another preferred embodiment, Ring A is 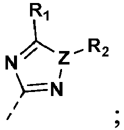 wherein R₁ is selected from the group consisting of H, substituted or unsubstituted C₁₋₆ alkyl, substituted or unsubstituted C₁₋₆ hydroxyalkyl, substituted or unsubstituted -C₁₋₄ alkylene-O-C₁₋₆ alkyl, substituted or unsubstituted -C₁₋₄ alkylene-S-C₁₋₆ alkyl;
Z is selected from the group consisting of O, S, and N;
when Z is O or S, R₂ is absent; when Z is N, R₂ is selected from the group consisting of H, and substituted or unsubstituted C₁₋₆ alkyl.

In another preferred embodiment, R₃ is substituted or unsubstituted C₁₋₆ alkoxy; preferably, R₃ is C₁₋₆alkoxy; more preferably, R₃ is methoxy.

In another preferred embodiment, R₄ is absent.

In another preferred embodiment, W₁ₐ and W_{1b} are each independently selected from the group consisting of -O-, -S-, -SO₂₋, -CO-, -NR₅-.

In another preferred embodiment, at most one of W₁ₐ and W_{1b} is -O-, i.e., -W₁ₐ-W_{1b}- is not -O-O-.

In another preferred embodiment, -W₁ₐ-W_{1b}- is -NR₅-CO-; preferably, is -NH-CO-.

In another preferred embodiment, W₂ₐ and W_{2b} are each independently selected from the group consisting of -O-, -S-, -SO₂₋, -CO-, -NR₅-.

In another preferred embodiment, at most one of W₂ₐ and W_{2b} is -O-, i.e., -W₂ₐ-W_{2b}- is not -O-O-.

In another preferred embodiment, -W₂ₐ-W_{2b}- is -NR₅- CO-; preferably, is -NH-CO-.

In another preferred embodiment, the compound is of formula Aa or formula Ab

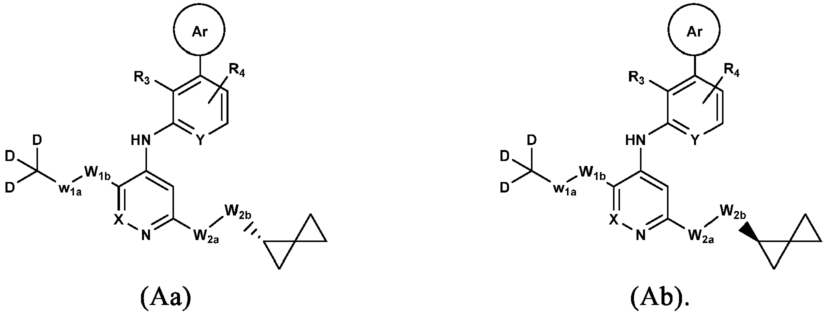

In another preferred embodiment, the compound is of formula I 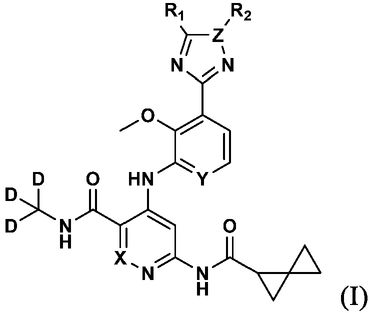 wherein,
R₁ is selected from the group consisting of H, substituted or unsubstituted C₁₋₆ alkyl, substituted or unsubstituted C₁₋₆ hydroxyalkyl, substituted or unsubstituted C₁₋₄ alkylene-O-C₁₋₆ alkyl, substituted or unsubstituted C₁₋₄ alkylene-S-C₁₋₆ alkyl;
Z is selected from the group consisting of O, S, and N;
when Z is O or S, R₂ is absent; when Z is N, R₂ is selected from the group consisting of H, and substituted or unsubstituted C₁₋₆ alkyl;
Y is CH or N;
X is CH or N;
unless otherwise specified, said substituted means that one or more (such as 1, 2, or 3) hydrogen in the group are substituted with substituents selected from the group consisting of halogen, C₁₋₄ alkyl, and C₁₋₄ haloalkyl.

In another preferred embodiment, Z is selected from the group consisting of O and N.

In another preferred embodiment, when Z is O or S, R₂ is absent; when Z is N, R₂ is substituted or unsubstituted C₁₋₆ alkyl (preferably, is methyl).

In another preferred embodiment, R₁ is substituted or unsubstituted C₁₋₄alkylene-O-C₁₋₆alkyl; preferably, R₁ is -CH₂OCH₃.

In another preferred embodiment, R₁ is H; Z is N; and R₂ is selected from the group consisting of H, substituted or unsubstituted C₁₋₆alkyl (preferably, R₂ is C₁₋₆alkyl; more preferably, R₂ is methyl).

In another preferred embodiment, R₁ is selected from the group consisting of substituted or unsubstituted C1-6alkyl, substituted or unsubstituted C₁₋₆hydroxyalkyl, substituted or unsubstituted C₁₋₄alkylene-O-C₁₋₆alkyl, substituted or unsubstituted C₁₋₄alkylene-S-C₁₋₆alkyl (preferably, R₁ is substituted or unsubstituted C₁₋₄alkylene-O-C₁₋₆alkyl; more preferably, R₁ is -CH₂OCH₃); Z is selected from the group consisting of O, and S (preferably, Z is O); R₂ is absent.

In another preferred embodiment, Y is CH or N.

In another preferred embodiment, X is CH or N.

In another preferred embodiment, the compound is of formula Ia or formula Ib

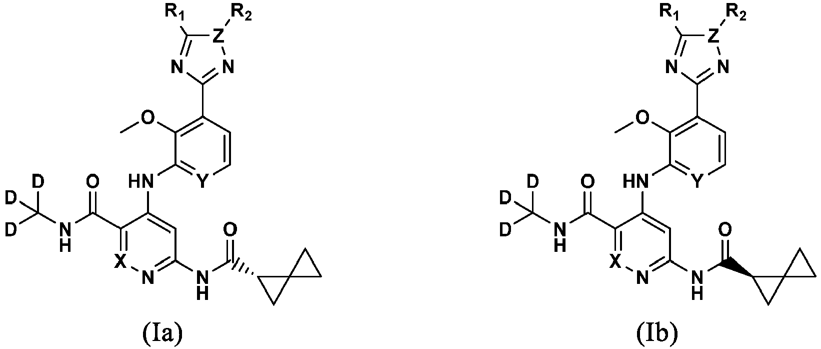

In another preferred embodiment, the compound is a compound selected from Table A:

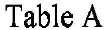

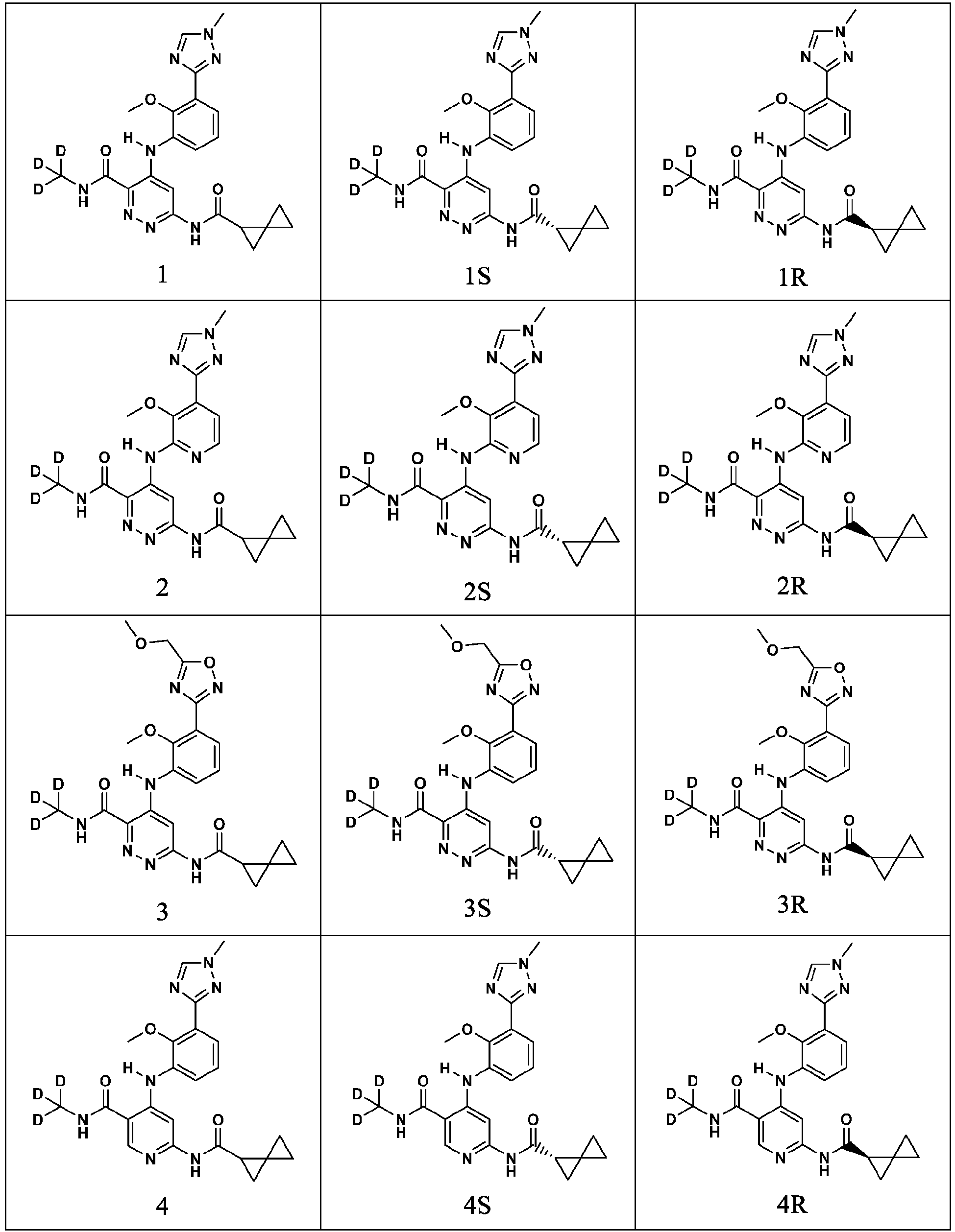

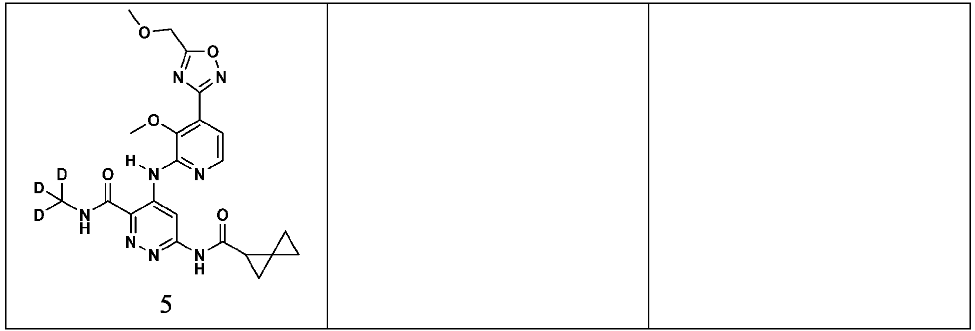

In the second aspect of the present invention, provided is a pharmaceutical composition, comprising
(i) the compound according to the first aspect or the pharmaceutically acceptable salt thereof, and (ii) pharmaceutically acceptable carriers or excipients.

In the third aspect of the present invention, provided is a use of the compound according to the first aspect or the pharmaceutical composition according to the second aspect in the preparation of (i) a drug for treating or preventing a TYK2 and/or JAK1-mediated disease and/or (ii) a TYK2 and/or JAK1 inhibitor.

In another preferred embodiment, TYK2 and/or JAK1 means TYK2 or JAK1 or a combination of the two.

In another preferred embodiment, the TYK2 and/or JAK1-mediated disease is a TYK2-mediated disease or a JAK1-mediated disease or a disease co-mediated by TYK2 and JAK1.

In another preferred embodiment, the TYK2 and/or JAK1 inhibitor is a TYK2 inhibitor or a JAK1 inhibitor or a TYK2 and JAK1 inhibitor.

In another preferred embodiment, the TYK2 and/or JAK1-mediated disease is a disease associated with IL-23, IL-12 and/or IFNα.

In another preferred embodiment, the compound treats or prevents the TYK2 and/or JAK1-mediated disease by selectively inhibiting TYK2-JH2 and/or JAK-JH2.

In another preferred embodiment, the TYK2 and/or JAK1-mediated disease comprises inflammation, autoimmune diseases, or combinations thereof.

In another preferred embodiment, the TYK2 and/or JAK1-mediated disease comprise psoriasis, lupus erythematosus, inflammatory bowel disease, psoriatic arthritis, arthritis, dermatitis, lupus nephritis, neuroinflammation such as multiple sclerosis and senile dementia, ankylosing spondylitis, hidradenitis suppurativa, respiratory disease, diabetes, inflammatory eye disease, hepatitis, cardiovascular disease, systemic sclerosis, organ transplantation, alopecia areata, acne, eczema, vitiligo, sjogren's syndrome, viral inflammation, cancer or combinations thereof.

In another preferred embodiment, the TYK2 and/or JAK1 inhibitor is a selective TYK2-JH2 and/or JAK-JH2 inhibitor.

In the fourth aspect of the present invention, provided is a method for selectively inhibiting TYK2 and/or JAK1, comprising:
contacting the subject with the compound according to the first aspect, thereby inhibiting the activity of TYK2 and/or JAK1 in the subject by TYK2-JH2 and/or JAK1-JH2.

In another preferred embodiment, the subject is cell.

In another preferred embodiment, the subject is TYK2 Kinase and/or JAK1 Kinase.

In another preferred embodiment, the inhibition is selective inhibition of TYK2-JH2 and/or JAK1-JH2.

In another preferred embodiment, the method is *in vitro* and non-therapeutic.

In the fifth aspect of the present invention, provided is a method for treating or preventing TYK2 and/or JAK1-mediated disease, comprising the steps of:
administering a safe and effective amount of the compound according to the first aspect or the pharmaceutical composition according to the second aspect to a human in need thereof, thereby treating or preventing the TYK2 and/or JAK1-mediated disease.

In another preferred embodiment, said TYK2 and/or JAK1-mediated disease is a disease associated with IL-23, IL-12 and/or IFNα.

In another preferred embodiment, the compound treats or prevents the TYK2 and/or JAK1-mediated disease by selectively inhibiting TYK2-JH2 and/or JAK1-JH2.

In another preferred embodiment, the TYK2 and/or JAK1-mediated disease comprises inflammation, autoimmune diseases, or combinations thereof.

In another preferred embodiment, the TYK2 and/or JAK1-mediated disease comprise: psoriasis, lupus erythematosus, inflammatory bowel disease, psoriatic arthritis, arthritis, dermatitis, lupus nephritis, neuroinflammation such as multiple sclerosis and senile dementia, ankylosing spondylitis, hidradenitis suppurativa, respiratory disease, diabetes, inflammatory eye disease, hepatitis, cardiovascular disease, systemic sclerosis, organ transplantation, alopecia areata, acne, eczema, vitiligo, sjogren's syndrome, cancer, viral inflammation, or combinations thereof.

In the sixth aspect of the present invention, provided is a method for preparing a compound of formula I according to the first aspect, the method comprises a step of:
reacting an intermediate of formula II with 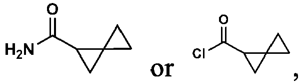 thereby obtaining the compound;

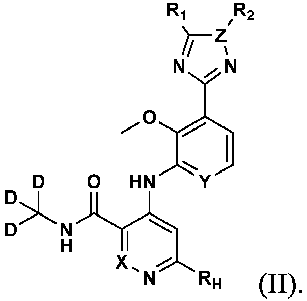

It should be understood that within the scope of the present invention, the above technical features of the present invention and the technical features specifically described in the following (e.g/, embodiments) can be combined with each other, thereby forming a new or preferred technical solution. Due to space limitations, it will not be repeated herein.

### EMBODIMENTS FOR CARRYING OUT THE INVENTION

After extensive and in-depth research, the inventor unexpectedly discovered a class of compounds with specific novel structures, i.e., compounds with a five-membered heteroaryl (ring Ar) and a structure of 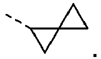 The compound has excellent selective inhibitory activity against the TYK2 pseudokinase (JH2) and the JAK1 pseudokinase (JH2) domain. Based on this, the inventors completed the present invention.

### TERMS

As used herein, the term "halogen" refers to F, Cl, Br or I. Correspondingly, "halo" means one or more hydrogen in the group is substituted with F, Cl, Br, or I.

Unless otherwise stated, the term "alkyl", by itself or as part of another substituent, means, a straight or branched chain hydrocarbon radical, having a specified number of carbon atoms (i.e. C₁₋₆ means 1 to 6 carbons). Examples of alkyl groups include methyl, ethyl, n-propyl, isopropyl, n-butyl, t-butyl, iso-butyl, sec-butyl, n-pentyl, n-hexyl, and the like.

The term "cycloalkyl" refers to a hydrocarbon ring with a specified number of ring atoms (for example, C₃₋₆ cycloalkyl has 3-6 ring atoms) and is completely saturated. "Cycloalkyl" is also meant to refer to bicyclic and polycyclic hydrocarbon rings, for example, bicyclo[2.2.1]heptane, bicyclo[2.2.2]octane, etc.

The term "alkylene" by itself or as part of another substituent means a divalent radical derived from an alkane, as exemplified by -CH₂-, -CH₂CH₂-, -CH(CH₃)-, -CH(CH₃)CH₂- or -CH₂CH₂CH₂-. Alkyl (or alkylene) typically has 1-4 carbon atoms. Similarly, "alkenylene" and "alkynylene" refer to the unsaturated forms of "alkylene" having a double bond or triple bond, respectively.

Unless otherwise specified, as used herein, the term "heteroatom" is intended to include oxygen (O), nitrogen (N), sulfur (S) and silicon (Si).

For the compounds provided herein, a bond that is drawn from a substituent (typically an R group) to the center of a ring (e.g. aromatic ring such as benzene, pyridine, and the like) will be understood to refer to a bond providing a connection at any of the available vertices of the ring.

As used herein, the term "containing", "comprising(comprise)" or "including" means that the various components can be used together in the mixture or composition of the present invention. Therefore, the terms "mainly consisting of ..." and "consisting of ..." are within the term "comprising".

As used herein, the term "pharmaceutically acceptable" component(s) refer to a substance suitable for use in human and/or animals without causing excessive adverse side reactions (such as toxicity, stimulation and allergic reaction), i.e. a substance with reasonable benefit/risk ratio.

Unless otherwise specified, all compounds present in the present invention are intended to include all possible optical isomers, such as single chiral compounds or mixtures of various chiral compounds (i.e. racemes). Among all compounds of the present invention, each chiral carbon atom may optionally be of the R or S configuration, or a mixture of the R and S configurations.

Certain compounds of the present invention possess asymmetric carbon atoms (optical centers) or double bonds; the racemates, diastereomers, geometric isomers, regioisomers and individual isomers (e.g., separate enantiomers) are all intended to be encompassed within the scope of the present invention. When compounds are provided herein with an identified stereochemistry (indicated as R or S, or with dashed or wedge bond designations), those compounds will be understood by those skilled in the art to be substantially free of other isomers (e.g., at least 80%, 90%, 95%, 98%, 99%, and up to 100% free of the other isomer).

The compounds of the present invention may also contain unnatural proportions of atomic isotopes at one or more of isotopic atoms that constitute such compounds. The unnatural proportions of certain isotope can be defined as the amount found naturally from the the atom discussed to 100% of that atom. For example, the compounds may incorporate radioactive isotopes, such as tritium (³H), iodine-125 (¹²⁵I) or carbon-14 (¹⁴C), or non-radioactive isotopes, such as deuterium (²H) or carbon-13 (¹³C). Such isotopic variants may provide additional uses in addition to those described in this application. For instance, isotopic variants of the compounds of the invention may have additional utility, including but not limited to, as diagnostic and/or imaging reagents, or as cytotoxic/radiotoxic therapeutic agents. Additionally, isotopic variants of the compounds of the present invention can have altered pharmacokinetic and pharmacodynamic characteristics which can contribute to enhancing safety, tolerability or efficacy during treatment. All isotopic variations of the compounds of the present invention, whether radioactive or not, should be encompassed within the scope of the present invention.

### Active Ingredients

As used herein, the term "compound of the invention" refers to the compound of formula (A) or formula (I). This term also includes various crystalline forms, pharmaceutically acceptable salts, hydrates or solvates of the compound of formula (A) or formula (I).

Wherein, the term "pharmaceutically acceptable salts" refers to salts that are formed of the compound of the invention and acids or bases, and suitable for use as a drug. Pharmaceutically acceptable salts include inorganic salts and organic salts. A group of preferred salts are salts formed of the compound of the present invention and the acids. Acids suitable for forming salts include, but are not limited to, inorganic acids such as hydrochloric acid, hydrobromic acid, hydrofluoric acid, sulfuric acid, nitric acid, phosphoric acid and the like; organic acids such as methanoic acid, acetic acid, trifluoroacetic acid, propionic acid, oxalic acid, malonic acid, succinic acid, fumanic acid, maleic acid, lactic acid, malic acid, tartaric acid, citric acid, bitter acid, benzoic acid, methanesulfonic acid, ethanesulfonic acid, p-toluenesulfonic acid, benzenesulfonic acid, naphthalene sulfonic acid, and the like; and amino acids such as proline, phenylalanine, aspartic acid, glutamic acid and the like. Another preferred type of salts are salts formed by compounds of the present invention and bases, e.g., alkali metal salts (e.g. sodium or potassium salts), alkaline earth metal salts (e.g. calcium or magnesium salts), ammonium salts (e.g., lower alkanol ammonium salts or other pharmaceutically acceptable amine salts), for example, methylamine salt, ethylamine salt, propylamine salt, dimethylamine salt, trimethylamine salts, diethylamine salts, triethylamine salts, tert-butyl amine salts, ethylenediamine salts, hydroxyethylamine salts, bi-hydroxyethylamine salts, tri-hydroxyethylamine salts, and amine salts formed by morpholine, piperazine, and lysine.

The term "solvate" refers to complexes formed by coordination of the compound of the present invention with solvent molecules in any specific ratio. The "hydrate" refers to complexes formed by coordination of the compound of the invention with water.

Moreover, the compound of the present invention further comprises prodrugs of the compounds of formula (A) or formula (I). The term "prodrug", including itself, is biologically active or non-active, when administered properly, readily undergo metabolism or chemical reaction in the human body and convert to the compound of formula (A) or formula (I), or the salts or solutions containing compound of formula (A) or formula (I). The prodrugs include (but are not limited to) the forms of carboxylate esters, carbonates, phosphates, nitrate, sulfates, sulfonyl esters, sulfoxide esters, amides, carbamates, azo compounds, phosphoramides, glucosides, ethers, acetals, and the like, of the compounds.

### Preparation method

Other parts herein provides a more specific description of the preparation methods for the compounds having a structure of formula (A) or formula (I) of the present invention, but these specific methods do not pose any limitations to the present invention. The compound of the present invention can also be conveniently prepared by combining various synthesis methods described in this specification or known in the art, and such combinations can be easily carried out by those skilled in this field to which the present invention belongs.

### pharmaceutical compositions and administration method

Because the compounds of the present invention have excellent selective inhibitory activity against TYK2 pseudokinase (JH2) and JAK1 pseudokinase (JH2) domain, the compounds of the invention and various crystal forms, pharmaceutically acceptable inorganic or organic salts, hydrates or solvates thereof, and pharmaceutical compositions containing the compound of the present invention as active ingredients can be used in the treatment, prevention and alleviation of diseases mediated by TYK2 kinase or JAK1 kinase or the both (i.e., TYK2 and/or JAK1-mediated disease). According to the prior art, the compounds of the invention can be used to treat the following diseases: various autoimmune and inflammatory related diseases including psoriasis, lupus erythematosus, inflammatory bowel disease, psoriatic arthritis, arthritis, dermatitis, lupus nephritis, neuroinflammation such as multiple sclerosis and senile dementia, ankylosing spondylitis, hidradenitis suppurativa, respiratory disease, diabetes, inflammatory eye disease, hepatitis, cardiovascular disease, systemic sclerosis, organ transplantation, alopecia areata, acne, eczema, vitiligo, sjogren's syndrome, viral inflammation, some cancers and the like.

As used herein, the term "selective" refers to that the activity or potency (such as inhibitory activity) against a specified target (such as TYK2 (JH2), JAK1(JH2)) is higher than the activity or potency (inhibitory activity) against other targets (such as JAK1, 2, 3 (JH1), and/or TYK2 (JH1)) ; for example, the activity or potency (such as inhibitory activity) against a specified target (such as TYK2 (JH2)) is at least 50 times of the activity or potency (such as inhibitory activity) against other targets (such as JAK1, 2, 3 (JH1), and/or TYK2 (JH1)). For example, when the IC50 value is used to quantify the inhibitory activity, IC50_{Other}/IC50_{TYK2/JAK1-JH2}>50, wherein IC50_{TYK2/JAK1-JH2} refers to the inhibitory activity IC50 (nM) of the compound against TYK2-JH2 (a pseudokinase domain of TYK2) or JAK1-JH2 (a pseudokinase domain of JAK1), and IC50_{Other} refers to the inhibitory activity IC50 (nM) of the compound against one or more of the following kinases: TYK2-JH1 (the kinase domain of TYK2), JAK1-JH1 (the kinase domain of JAK1), JAK2-JH1 (the kinase domain of JAK1), and JAK3-JH1 (the kinase domain of JAK3).

The pharmaceutical composition of the invention comprises the compound of the present invention or the pharmaceutically acceptable salts thereof in a safe and effective dosage range, and pharmaceutically acceptable excipients or carriers. Wherein, "safe and effective amount" refers to the amount of compound which is sufficient to significantly improve the condition, and not to generate severe side effects. Generally, the pharmaceutical composition contains 1-200mg of the compounds of the invention per dose, more preferably, 1-50mg of the compounds of the invention per dose. Preferably, the "one dose" is one capsule or one pill.

"Pharmaceutically acceptable carrier" means one or more compatible solid or liquid fillers, or gelatinous materials which are suitable for human use and should be of sufficient purity and sufficiently low toxicity. "Compatibility" used herein means that the components of the composition can be admixed with the compounds of the invention and with each other without significantly reducing the efficacy of the compounds. Some examples of pharmaceutically acceptable carriers include cellulose and the derivatives thereof (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween^{®}), wetting agent (such as sodium dodecyl sulfate), coloring agents, flavoring agents, stabilizers, antioxidants, preservatives, pyrogen-free water, etc.

There are no special limitations on administration mode of the compound or pharmaceutical compositions of the present invention, and the representative administration mode includes (but is not limited to): oral, intratumoral, rectal, parenteral (intravenous, intramuscular or subcutaneous), and topical administration.

Solid dosage forms for oral administration include capsules, tablets, pills, powders and granules. In these solid dosage forms, the active compounds are mixed with at least one conventional inert excipient (or carrier), such as sodium citrate or Ca₂HPO₄, or mixed with the following components: (a) fillers or compatibilizer, for example, starch, lactose, sucrose, glucose, mannitol and silicic acid; (b) binders, for example, hydroxymethyl cellulose, alginates, gelatin, polyvinylpyrrolidone, sucrose and arabic gum; (c) humectant, such as, glycerol; (d) disintegrating agents, such as agar, calcium carbonate, potato starch or tapioca starch, alginic acid, certain composite silicates, and sodium carbonate; (e) retarding solvents, such as paraffin; (f) absorption accelerators, for example, quaternary ammonium compounds; (g) wetting agents, such as cetyl alcohol and glyceryl monostearate; (h) adsorbents, for example, kaolin; and (i) lubricants, such as talc, stearin calcium, magnesium stearate, solid polyethylene glycol, sodium lauryl sulfate, or the mixtures thereof. In capsules, tablets, and pills, the dosage form may also include a buffer.

Solid dosage forms such as tablets, sugar pills, capsules, pills, and granules can be prepared using coating and shell materials, such as casings and other well-known materials in the art. They can contain opaque agents. The release of the active compounds or compounds in the compositions can be released in a delayed manner in a given portion of the digestive tract. Examples of embedding components that can be used are polymeric substances and waxes. If necessary, the active compound can also form microcapsules with one or more of the aforementioned excipients.

Liquid dosage forms for oral administration include pharmaceutically acceptable emulsions, solutions, suspensions, syrups or tinctures. In addition to the active compounds, the liquid dosage forms may contain any conventional inert diluents known in the art such as water or other solvents, solubilizers and emulsifiers, for example, ethanol, isopropanol, ethyl carbonate, ethyl acetate, propylene glycol, 1,3-butanediol, dimethyl formamide, as well as oil, in particular, cottonseed oil, peanut oil, corn germ oil, olive oil, castor oil and sesame oil, or the combination thereof.

In addition to these inert diluents, the composition can also include additives such as wetting agents, emulsifiers and suspending agents, sweeteners, corrigents, and spices.

In addition to active compounds, suspensions can include suspending agents such as ethoxylated isooctadecanol, polyoxyethylene sorbitol and dehydrated sorbitol esters, microcrystalline cellulose, methanol aluminum and agar, or mixtures of these substances.

The compositions for parenteral injection may comprise physiologically acceptable sterile aqueous or anhydrous solutions, dispersions, suspensions or emulsions, and sterile powders which can be re-dissolved into sterile injectable solutions or dispersions. Suitable aqueous and non-aqueous carriers, diluents, solvents or excipients include water, ethanol, polyols, and their suitable mixtures.

The dosage forms of the compounds of the present invention used for local administration include ointments, powders, patches, sprays, and inhalants. The active ingredients are mixed under sterile conditions with a physiologically acceptable carriers and any preservatives, buffers, or propellants if necessary.

The compound of the present invention can be administered separately or in combination with other pharmaceutically acceptable compounds.

When the pharmaceutical compositions are used, a safe and effective amount of the compound of the present invention is administered to a mammal (such as human) in need of, wherein the dose for administration is a pharmaceutically effective dose. For a person weighed 60 kg, the daily dose is usually 1- 200mg, preferably 1 -50 mg. Of course, the specific dosage should also consider factors such as the route of administration and the patient's health status, which are within the skill range of a skilled physician.

### The main advantages of the present invention include:

1. The compound of the present invention has excellent selectivity. Compared with non-selective JAK inhibitors, the compounds of the present invention have excellent inhibitory activity against the pseudokinase domains (JH2) of TYK2 and JAK1, while not inhibiting the JH1 region that has kinase action function. Therefore, the TYK2/JAK1 pseudokinase domain (JH2) selective inhibitor of the present invention has superior safety.
2. The compound of the present invention has excellent selectivity as well as excellent inhibitory activity.

The present invention was further described hereafter in combination with specific embodiments. It should be understood that these examples are only used to illustrate the present invention and not to limit the scope of the invention. The experimental methods in the following examples that do not specify specific conditions generally follow the conventional conditions or the conditions suggested by the manufacturer. Unless otherwise stated, percentages and parts are percentages by weight and parts by weight.

### Preparation Example

### Example 1:

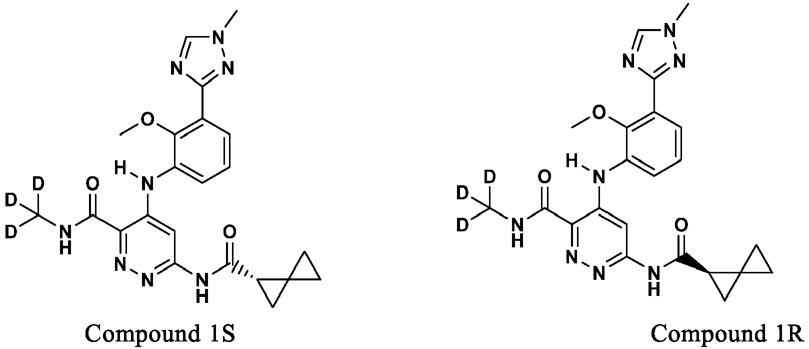

### STEP A

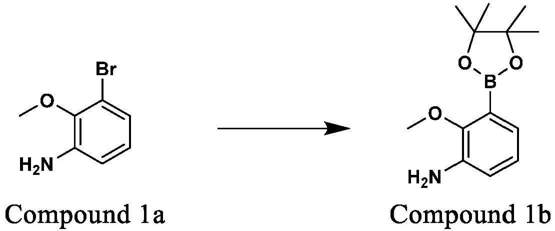

Compound 1a (1.3g, 6.4mmol) was dissolved in dioxane (35mL). The solution was successively added with Bis(pinacolato)diboron (2.44g, 9.6mmol), potassium acetate (1.26g, 12.8mmol) and a complex of [1,1'-bis(diphenylphosphino)ferrocene]palladium(II) dichloride and dichloromethane (0.53g, 0.64mmol). The mixture was stirred at 90°C for 5 hours under nitrogen protection. The reaction was monitored by LCMS. After the completion of reaction, the reaction was cooled to room temperature, and concentrated under reduced pressure to obtain crude product. The crude product was separated by rapid preparative chromatograph (eluent: petroleum ether/ethyl acetate, 2/1, v/v) under medium pressure to obtain Compound 1b (680mg, 43.4%) as yellow solid. 1HNMR (400 MHz, DMSO-d6): δ ppm 6.79 - 6.75. (m, 3H), 4.80 (s, 2H), 3.63 (s, 3H), 1.28 (s, 12H). MS: 249.9 [M+H]+.

### STEP B

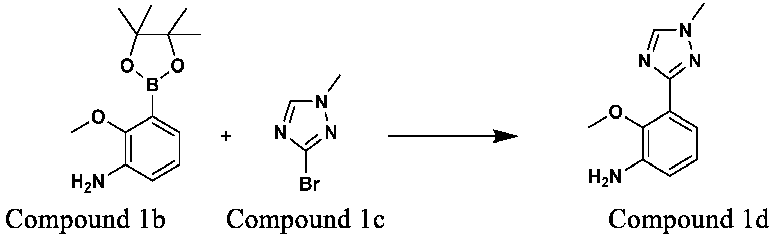

Compound 1b (330mg, 1.32mmol) was dissolved in dioxane (16mL) and water (4mL), and the solution was successively added with Compound 1c (233mg, 1.45mmol), tetrakis(triphenylphosphine)palladium (150mg, 0.13mmol) and cesium carbonate (863mg, 2.65mmol). The mixture was stirred at 90°C for 6 hours under nitrogen protection. The reaction was monitored by LCMS. After the completion of reaction, the reaction was cooled to room temperature, diluted with water (20 mL) and extracted with ethyl acetate (30 mL × 2). The extracts were combined, washed with saturated saline, dried over anhydrous sodium sulfate and concentrated under reduced pressure to obtain crude product. The crude product was separated by rapid preparative chromatograph (eluent: petroleum ether/ethyl acetate, 1/1, v/v) under medium pressure to obtain Compound 1d (230 mg, 84.5%) as yellow solid. 1H NMR (400 MHz, DMSO-d6): δ ppm 8.47 (s, 1H), 6.96 - 6.94 (m, 1H), 6.86 (t, J = 8.0 Hz, 1H), 6.75 - 6.73 (m, 1H), 4.98 (s, 2H), 3.91 (s, 3H), 3.66 (s, 3H). MS: 205.2 [M+H]+.

### STEP C

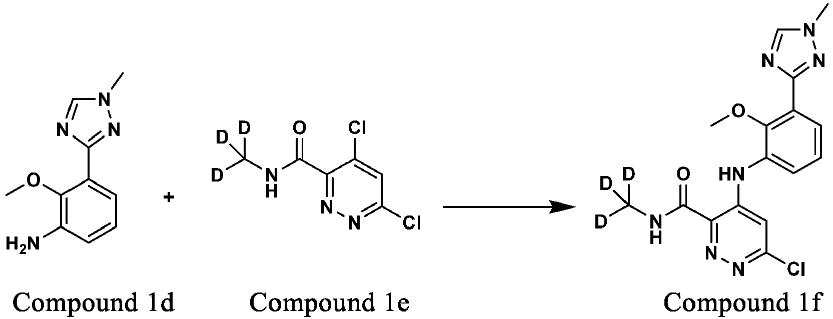

Compound 1d (210 mg, 1.03 mmol) and Compound 1e (258 mg, 1.23 mmol) were dissolved in dry tetrahydrofuran (20 mL). Di-trimethylsilylamine lithium (1M in THF, 3.1 mL, 3.1 mmol) was added dropwise at 0°C. The mixture was stirred at room temperature for 16 hours under nitrogen protection. The reaction was monitored by LCMS. After the completion of reaction, the reaction was quenched by adding water (20 mL) and extracted with ethyl acetate (30 mL × 2). The extracts were combined, washed with saturated saline, dried over anhydrous sodium sulfate and concentrated under reduced pressure to obtain crude product. The crude product was separated by rapid preparative chromatograph (dichloromethane/methanol, 10/1, v/v) under medium pressure to obtain Compound 1f (340 mg, 87.8%) as yellow solid. MS: 377.2 [M+H]+.

### STEP D:

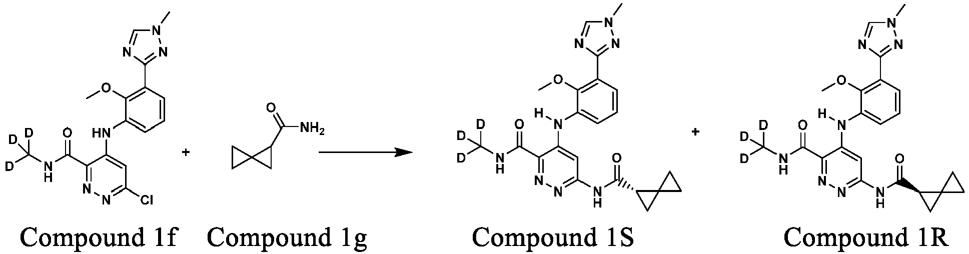

Compound 1f (310mg,0.82mmol) and Compound 1g (183mg,1.64mmol) were dissolved in dioxane (30mL). The solution was successively added with XantPhos (142mg,0.24mmol), cesium carbonate (536mg,1.64mmol) and XPhosPdG2 (65mg,0.08mmol). The mixture was stirred at 110°C for 16 hours under nitrogen protection. The reaction was monitored by LCMS. After the completion of reaction, the reaction was diluted with water (20 mL) and extracted with ethyl acetate (20 mL × 3). The extracts were combined, washed with saturated saline (20mL), dried over anhydrous sodium sulfate and concentrated under reduced pressure. The residue was separated by rapid preparative chromatograph (dichloromethane/methanol, 10/1-5/1, v/v) under medium pressure to obtain 105mg crude product. The crude product was separated via SFC (Thar prep 80, Column: CHIRALPAK AD-H 250 mm × 20 mm × 5µm, modifier: 40% methanol (NH4OH 0.2%)/60% CO2, Total flow rate: 40 g/min, Temperature: 40°C) to obtain two isomers: Compound 1S(40 mg, Rt: 8.85 min, OR: 203.4, 24.5°C), and Compound 1R (44 mg, Rt: 13.81 min, OR: -237.6, 24.5°C) total yield: 22.6%.

**Compound 1S:** 1H NMR (400 MHz, CD3OD): δ ppm 11.10 (s, 1H), 10.96 (s, 1H), 9.10 (s, 1H), 8.53 (s, 1H), 8.14 (s, 1H), 7.64 - 7.61 (m, 1H), 7.50 - 7.48 (m, 1H), 7.28 - 7.24 (m, 1H), 3.91 (s, 3H), 3.69 (s, 3H), 2.49 - 2.38 (m, 1H), 1.36 - 1.29 (m, 2H), 0.87 - 0.69 (m, 4H). MS: 452.2 [M+H]+.

**Compound 1R:** 1H NMR (400 MHz, CD3OD): δ ppm 11.10 (s, 1H), 10.96 (s, 1H), 9.10 (s, 1H), 8.53 (s, 1H), 8.14 (s, 1H), 7.64 - 7.61 (m, 1H), 7.50 - 7.48 (m, 1H), 7.28 - 7.24 (m, 1H), 3.91 (s, 3H), 3.69 (s, 3H), 2.49 - 2.38 (m, 1H), 1.36 - 1.29 (m, 2H), 0.87 - 0.69 (m, 4H). MS: 452.2 [M+H]+.

### Example 2:

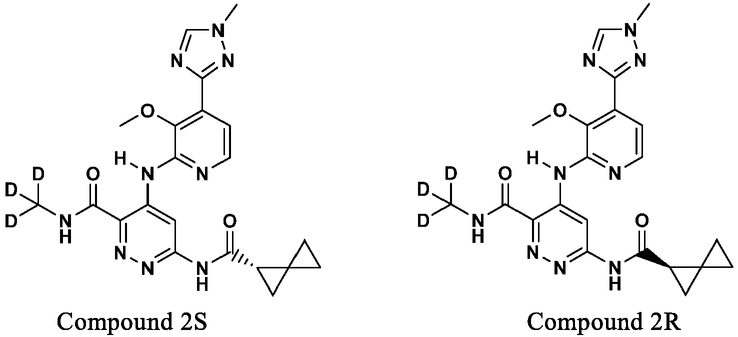

### STEP A

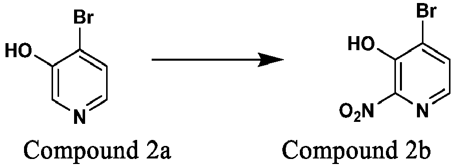

Compound 2a (10 g, 57.82 mmol) was added to a three-necked flask containing 200 ml concentrated sulfuric acid at 0°C. The mixture was added with 90% fuming concentrated nitric acid (6 g, 86.21 mmol) at 0 °C under stirring, and stirred at room temperature for 20h. The reaction was monitored by LCMS. After the completion of reaction, the reaction solution was poured into 300mL ice water and the mixture was diluted with ethyl acetate (500 mL), washed with water (200 mL × 2), and saturated aqueous sodium chloride (200 mL × 2) respectively, dried over anhydrous Na2SO4, and concentrated under reduced pressure to obtain Compound 2b (7.8 g, 61.9%) as yellow solid. 1H NMR (400 MHz, CD3OD): δ ppm: 7.99 - 7.98 (m, 1H), 7.93 - 7.92 (m, 1H). MS: 219.0, 221.0[M+H]+

### STEP B

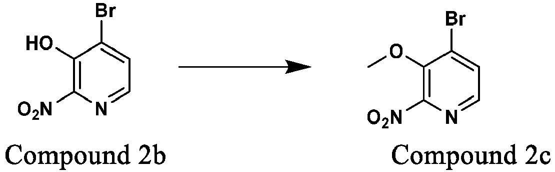

Compound 2b (7.8 g, 35.79 mmol), and potassium carbonate (9.8 g, 71.24 mmol) were successively added to a 250mL round bottom flask containing 100 mL N,N-dimethylformamide. The mixture was stirred at room temperature for 10 min and added with methyl iodide (10.1 g, 71.24 mmol) and then stirred at 30°C for 20h. The reaction was monitored by LCMS. After the completion of reaction, the reaction was filtered, and the filtrate was concentrated under reduced pressure. The residue was added with 100 mL water, and extracted with ethyl acetate (100 mL × 3). The extracts were combined, dried over anhydrous Na2SO4, and concentrated under reduced pressure. The residue was separated by column chromatograph (eluent: petroleum ether/ethyl acetate, 20/1, v/v) to obtain Compound 2c (4 g, 48.2%) as white solid. 1H NMR (400 MHz, CDCL3): δ ppm: 8.09 (d, J =5.2 Hz, 1H), 7.78 (d, J =5.2 Hz, 1H), 4.04 (s, 3H). MS: 233.0, 235.0[M+H]+

### STEP C

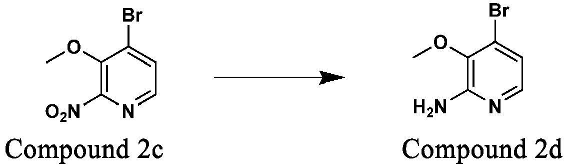

Compound 2c(4 g, 17.2 mmol), and iron powder (4.8 g, 86 mmol) were successively added to a 100mL round bottom flask containing 20 mL ethanol/20 mL acetic acid/10 mL water. The mixture was stirred at room temperature for 2h. The reaction was monitored by LCMS. After the completion of reaction, the reaction was filtered, and the filtrate was concentrated under reduced pressure. The residue was added with 50 mL water, adjusted to pH=10 using 2 N sodium hydroxide solution, and extracted with dichloromethane (50 mL × 3). The extracts were combined, dried over anhydrous Na2SO4 and concentrated under reduced pressure. The residue was separated by column chromatograph (dichloromethane/methanol, 20/1, v/v) to obtain Compound 2d (3g, 86.0%) as yellow solid. 1H NMR (400 MHz, CDCL3): δ ppm: 7.60 (d, J =5.2 Hz, 1H), 6.82 (d, J =5.6 Hz, 1H), 4.99 (s, 2H), 3.85 (s, 3H). MS: 203.0, 205.1 [M+H]+

### STEP D

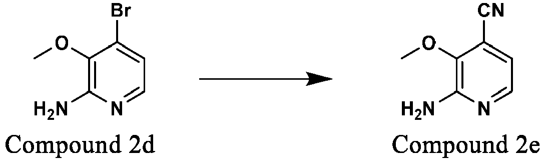

Compound 2d (2 g, 9.9 mmol), zinc cyanide (1.28 g, 10.8 mmol), 1,1 '- bis (diphenylphosphine) ferrocene (1.05 g, 1.9 mmol) and zinc powder (58.5 mg, 0.9 mmol) were added to a 100 mL round bottom flask containing N,N-dimethylformamide (40 mL). Then tris(dibenzylideneacetone)dipalladium (0.91 g, 0.9 mmol) was added. The mixture was stirred at 130°C for 16 hours under nitrogen protection. The reaction was monitored by LCMS. After the completion of reaction, the reaction was diluted with water (300 mL) and filtered. The filtrate was extracted with ethyl acetate (300 mL × 2). The extracts were combined, washed with saturated saline, dried over anhydrous sodium sulfate, and concentrated under reduced pressure. The residue was separated by rapid preparative chromatograph (petroleum ether/ethyl acetate, 1/1, v/v) under medium pressure to obtain Compound 2e (0.98 g, 65.9%) as yellow solid. 1H NMR (400 MHz, CD3OD): δ ppm 7.71. (d, J=2.8 Hz, 1H), 6.70. (d, J=2.8 Hz, 1H), 4.01 (s, 3H). MS: 150.0 [M+H]+.

### STEP E

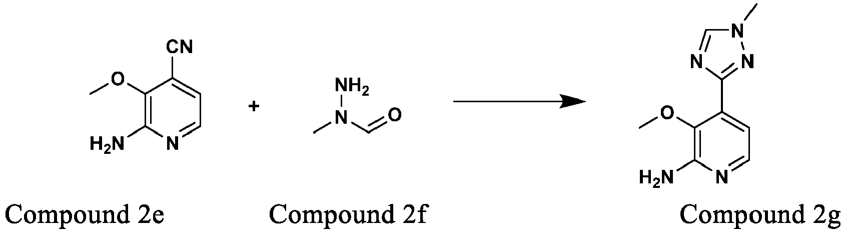

Compound 2e (0.5 g, 3.35 mmol), Compound 2f (745 mg, 10.05 mmol) and potassium tert-butoxide (1.5 g, 13.4 mmol) were added to xylene (20 mL) all at once. The mixture was stirred at 140°C for 3 hours under nitrogen protection. The reaction was monitored by LCMS. After the completion of reaction, the reaction was cooled to room temperature, and concentrated under reduced pressure to obtain crude product. The crude product was separated by rapid preparative chromatograph (dichloromethane/methanol, 10/1, v/v) under medium pressure to obtain Compound 2g (60 mg, 8.7%) as yellow solid. MS: 205.9 [M+H]+.

### STEP F

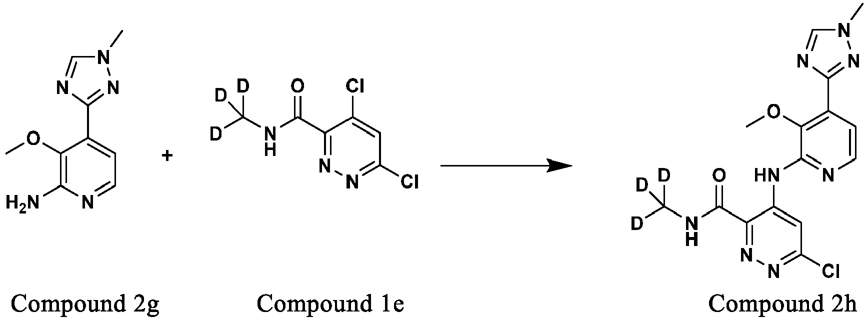

Compound 2g (60 mg, 0.29 mmol) and Compound 1e (73 mg, 0.35 mmol) were dissolved in dry tetrahydrofuran (20 mL). Di-trimethylsilylamine lithium (1M in THF, 0.88 mL, 0.88 mmol) was added dropwise at -78 °C. The mixture was stirred at room temperature for 16 hours under nitrogen protection. The reaction was monitored by LCMS. After the completion of reaction, the reaction was quenched by adding water (20 mL) and extracted with ethyl acetate (30 mL × 2). The extracts were combined, washed with saturated saline, dried over anhydrous sodium sulfate and concentrated under reduced pressure to obtain crude product. The crude product was separated by rapid preparative chromatograph (dichloromethane/methanol, 10/1, v/v) under medium pressure to obtain Compound 2h (44 mg, 40.1%) as yellow solid. MS: 378.2 [M+H]+.

### STEP G

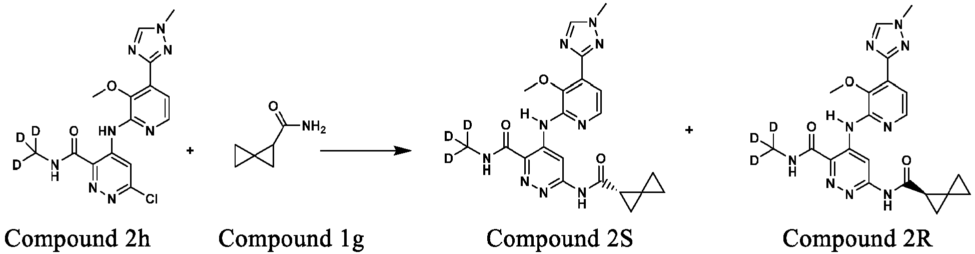

Compound 2h (44 mg, 0.11 mmol) and Compound 1g (38.8 mg, 0.35 mmol) were dissolved in dioxane (10 mL). The solution was successively added with XantPhos (13.5 mg, 0.023 mmol), cesium carbonate (75 mg, 0.23 mmol) and XPhos PdG2 (18.1 mg, 0.023 mmol). The mixture was stirred at 120°C for 16 hours under nitrogen protection. The reaction was monitored by LCMS. After the completion of reaction, the reaction was diluted with water (20 mL) and extracted with ethyl acetate (20 mL × 3). The extracts were combined, washed with saturated saline (20 mL), dried over anhydrous sodium sulfate and concentrated under reduced pressure. The residue was separated by rapid preparative chromatograph (dichloromethane/methanol, 10/1-5/1, v/v) under medium pressure to obtain 65 mg crude product. The crude product was separated via SFC (Thar prep 80, Column: CHIRALPAK AD-H 250 mm × 20 mm × 5µm, modifier: 40% methanol (NH4OH 0.2%)/60% CO2, Total flow rate: 40 g/min, Temperature: 40°C) to obtain two isomers: Compound 2S (5.2 mg, Rt: 8.85 min, OR: 235.9, 27.1°C), and Compound 2R (5.6 mg, Rt: 12.64 min, OR: -237.6, 27.1°C) total yield: 21.7%.

Compound 2S: 1H NMR (400 MHz, CD3OD): δ ppm 12.44(s, 1H), 11.15 (s, 1H), 9.89 (s, 1H), 9.24 (s, 1H), 8.67 (s, 1H), 8.17-8.16 (m, 1H), 7.51 - 7.50 (m, 1H), 4.00 (s, 3H), 3.90 (s, 3H), 2.49 - 2.47 (m, 1H), 1.47 - 1.38 (m, 2H), 0.92 - 0.80 (m, 4H). LCMS: 453.3 [M+H]+.

Compound 2R: 1H NMR (400 MHz, CD3OD): δ ppm 12.44(s, 1H), 11.15 (s, 1H), 9.89 (s, 1H), 9.24 (s, 1H), 8.67 (s, 1H), 8.17-8.16 (m, 1H), 7.51 - 7.50 (m, 1H), 4.00 (s, 3H), 3.90 (s, 3H), 2.49 - 2.47 (m, 1H), 1.47 - 1.38 (m, 2H), 0.92 - 0.80 (m, 4H). LCMS: 453.2 [M+H]+.

### Example 3:

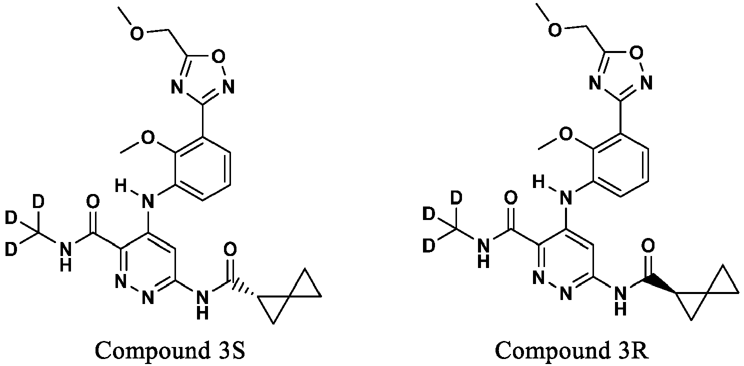

### STEP A

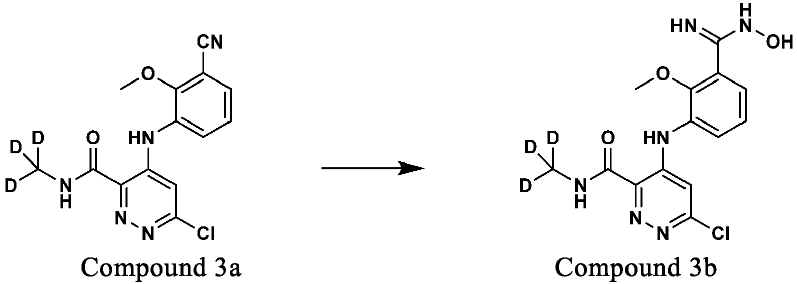

Compound 3a (1 g, 3.1 mmol) was added to ethanol (20 mL), followed by the addition of hydroxylamine aqueous solution (0.82 g, 50%, 12.4 mmol) and the mixture was stirred at 60°C for 16 hour. The reaction was monitored by LCMS. After the completion of reaction, the reaction was concentrated under reduced pressure. The residue was separated by rapid preparative chromatograph (dichloromethane/methanol, 10/1, v/v) under medium pressure to obtain Compound 3b (0.61 g, 55.7%) as yellow solid. MS: 353.7 [M+H]+.

### STEP B

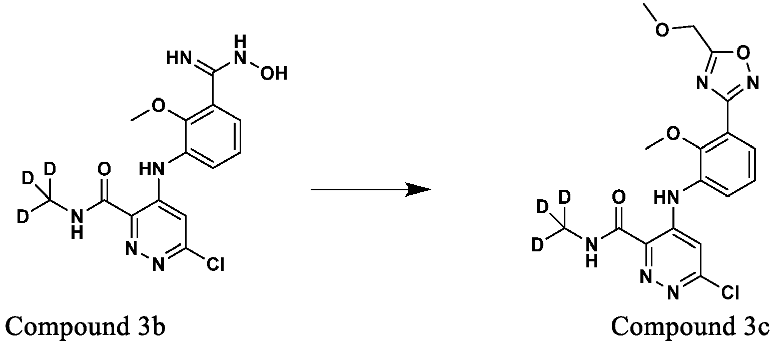

Compound 3b (300 mg, 0.85 mmol) and triethylamine (257 mg, 2.55 mmol) were added to dichloromethane (10 mL), and then methoxyacetyl chloride (184 mg, 1.7 mmol) was added dropwise. The mixture was stirred at room temperature for 3 hours under nitrogen protection. Then 1N tetrabutylammonium fluoride in tetrahydrofuran (3.4 mL, 3.4 mmol) was added. The reaction was monitored by LCMS. After the completion of reaction, the reaction was quenched by adding water (20 mL) and extracted with dichloromethane (30 mL × 2). The extracts were combined, washed with saturated saline, dried over anhydrous sodium sulfate and concentrated under reduced pressure to obtain crude product. The crude product was separated by rapid preparative chromatograph (dichloromethane/methanol, 10/1, v/v) under medium pressure to obtain Compound 3c (260 mg, 75.1%) as yellow solid. MS: 408.1 [M+H]+.

### STEP C

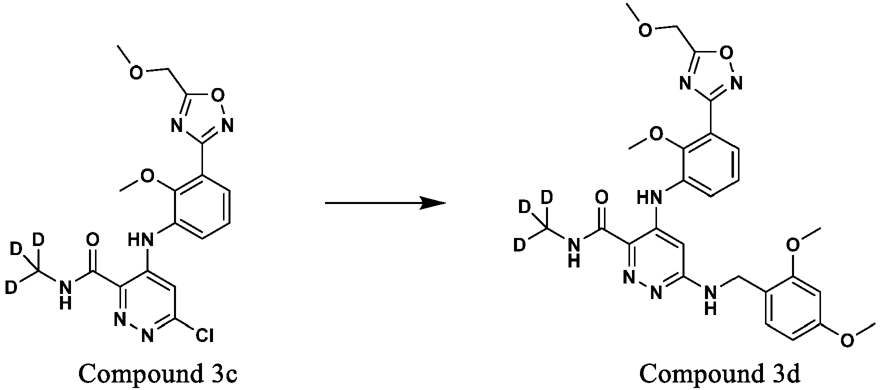

Compound 3c (260 mg, 0.64 mmol) and 2,4-dimethoxybenzylamine (213 mg, 1.28 mmol) were dissolved in dimethyl sulfoxide (10 mL), followed by the addition of potassium fluoride(110 mg, 19 mmol). The mixture was stirred at 110°C for 4 hours under nitrogen protection. The reaction was monitored by LCMS. After the completion of reaction, the reaction was quenched by adding water (20 mL) and extracted with ethyl acetate (30 mL × 2). The extracts were combined, washed with saturated saline, dried over anhydrous sodium sulfate and concentrated under reduced pressure to obtain crude product. The crude product was separated by rapid preparative chromatograph (dichloromethane/methanol, 10/1, v/v) under medium pressure to obtain Compound 3d (220 mg, 64.1%) as yellow solid. MS: 539.2 [M+H]+.

### STEP D

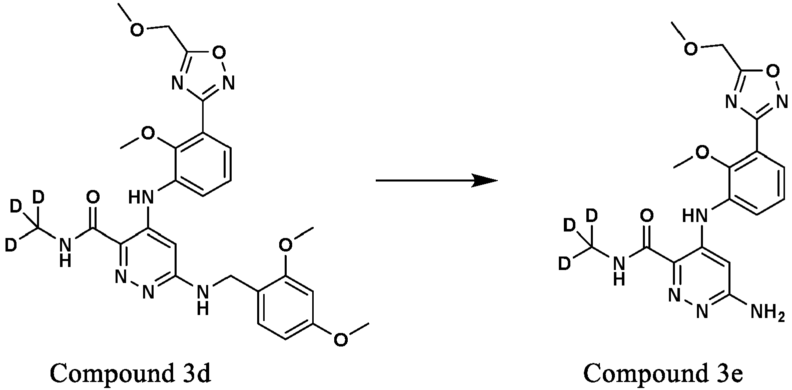

Compound 3d (220 mg, 0.41 mmol) was dissolved in dichloromethane (5 mL). Trifluoroacetic acid (5 mL) was added. The mixture was stirred at room temperature for 4 hours under nitrogen protection. The reaction was monitored by LCMS. After the completion of reaction, the reaction was concentrated under reduced pressure, adjusted to pH=8 with 1N sodium hydroxide solution, and extracted with ethyl acetate (30 mL × 2). The extracts were combined, washed with saturated saline, dried over anhydrous sodium sulfate and concentrated under reduced pressure to obtain crude product. The crude product was separated by rapid preparative chromatograph (dichloromethane/methanol, 10/1, v/v) under medium pressure to obtain Compound 3e (140 mg, 88.0%) as yellow solid. MS: 388.7 [M+H]+.

### STEP E

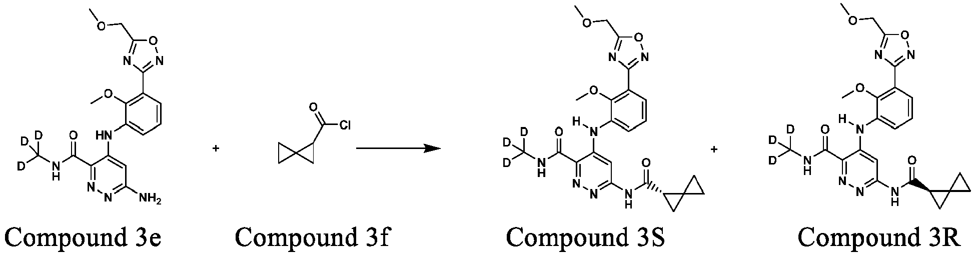

Compound 3e (110 mg, 0.28 mmol) and pyridine (67 mg, 0.85 mmol) were dissolved in 1,2-dichloroethane (10 mL). Then Compound 3f (111 mg, 0.85 mmol) was added dropwise. The mixture was stirred at 80°C for 16 hours under nitrogen protection. The reaction was monitored by LCMS. After the completion of reaction, the reaction was diluted with water (20 mL) and extracted with ethyl acetate (20 mL × 3). The extracts were combined, washed with saturated saline (20 mL), dried over anhydrous sodium sulfate and concentrated under reduced pressure. The residue was separated by rapid preparative chromatograph (eluent: dichloromethane/methanol, 10/1 - 5/1, v/v) under medium pressure to obtain 50 mg crude product. The crude product was separated via SFC (Thar prep 80, Column: CHIRALPAK AD-H 250 mm × 20 mm × 5µm, modifier: 40% methanol (NH₄OH 0.2%)/60% CO2, Total flow rate: 40 g/min, Temperature: 40°C)) to obtain two isomers: Compound 3S (12 mg, Rt: 2.60 min, OR: 143.4, 25.0°C), and Compound 3R (11 mg, Rt: 3.03 min, OR: -139.1, 25.0°C) total yield: 17.0%.

Compound 3S: 1H NMR (400 MHz, DMSO- d6): δ ppm 11.13(s, 1H), 11.04 (s, 1H), 9.13 (s, 1H), 8.15 (s, 1H), 7.71 - 7.66 (m, 2H), 7.40 - 7.36 (m, 1H), 4.82 (s, 2H), 3.72 (s, 3H), 3.42 (s, 3H), 2.41 - 2.39 (m, 1H), 1.36 - 1.30 (m, 2H), 0.84 - 0.69 (m, 4H). LCMS: 483.2 [M+H]+.

Compound 3R: 1H NMR (400 MHz, DMSO- d6): δ ppm 11.18(s, 1H), 11.08 (s, 1H), 9.18 (s, 1H), 8.19 (s, 1H), 7.73 - 7.70 (m, 2H), 7.44 - 7.40 (m, 1H), 4.86 (s, 2H), 3.75 (s, 3H), 3.45 (s, 3H), 2.45 - 2.43 (m, 1H), 1.38 - 1.34 (m, 2H), 0.90 - 0.73 (m, 4H). LCMS: 483.2 [M+H]+.

### Example 4:

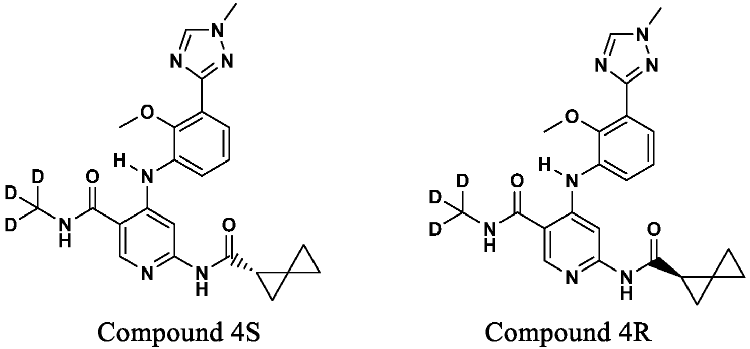

### STEP A

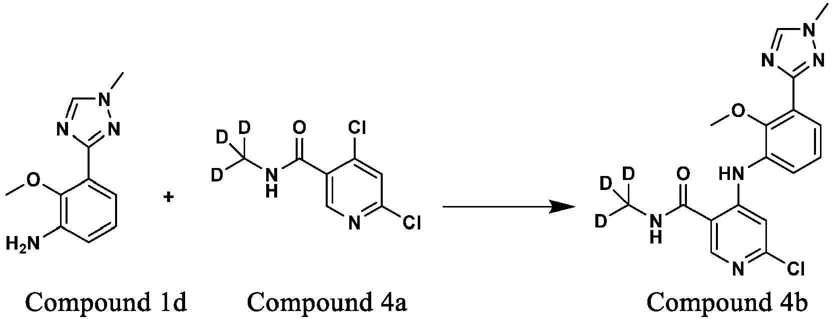

Compound 1d (500 mg, 2.45 mmol) was dissolved in dry tetrahydrofuran (20 mL). Di-trimethylsilylamine lithium (1M in THF, 7.4 mL, 7.4 mmol) was added dropwise at -78°C. The mixture was stirred at -78°C for 1 hour under nitrogen protection. Then compound 4a (561 mg, 2.71 mmol) was added, and the mixture was stirred at room temperature for 16 hours. The reaction was monitored by LCMS. After the completion of reaction, the reaction was quenched by adding water (20 mL) and extracted with ethyl acetate (30 mL × 2). The extracts were combined, washed with saturated saline, dried over anhydrous sodium sulfate and concentrated under reduced pressure. The residue was separated by rapid preparative chromatograph (dichloromethane/methanol, 10/1, v/v) under medium pressure to obtain Compound 4b (710 mg, 77.2%) as yellow solid. 1H NMR (400 MHz, CDCL3): δ ppm 10.63 (s, 1H), 8.71 (s, 1H), 8.14 (s, 1H), 7.88 (dd, J = 7.6, 1.6 Hz, 1H), 7.46 (s, 1H), 7.38 (dd, J = 7.8, 1.6 Hz, 1H), 7.27 - 7.25 (m, 1H), 6.90 (s, 1H), 4.02 (s, 3H), 3.81 (s, 3H). MS: 375.7, 377.7 [M+H]+.

### STEP B

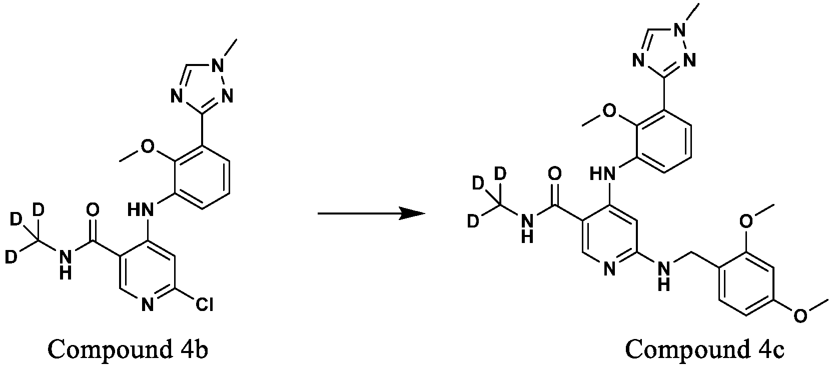

Compound 4b (370 mg, 0.99 mmol) and 2,4-dimethoxybenzylamine (329 mg, 1.97 mmol), and potassium fluoride were successively added to a round bottom flask containing 10 mL dimethyl sulfoxide. The mixture was stirred at 120°C for 48 hours. The reaction was monitored by LCMS. After the completion of reaction, the reaction was diluted with ethyl acetate (40 mL), washed with water (20 mL × 2) and saturated saline (20 mL × 2) respectively, dried over anhydrous sodium sulfate and concentrated under reduced pressure. The residue was separated by rapid preparative chromatograph (dichloromethane/methanol, 10/1, v/v) under medium pressure to obtain Compound 4c (290 mg, 58.2%) as yellow solid. MS: 506.6 [M+H]+.

### STEP C

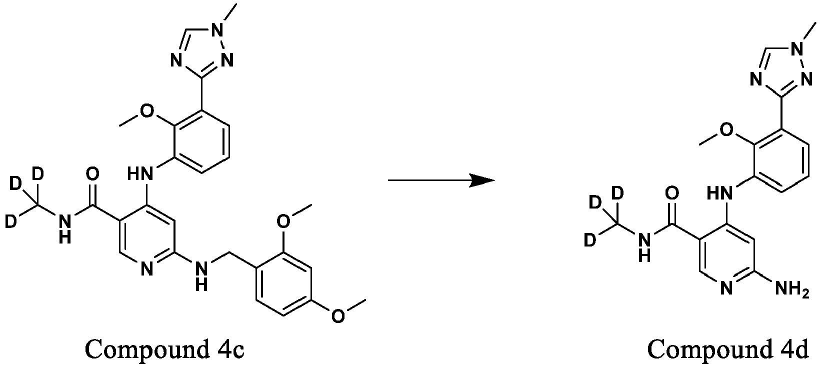

Compound 4c (270 mg, 0.53 mmol) was added to a round bottom flask containing 5 mL dichloromethane, and the mixture was added with trifluoroacetic acid (1 mL) dropwise, and stirred at room temperature for 2h. The reaction was monitored by LCMS. After the completion of reaction, the reaction was adjusted to pH=8 by adding 2N sodium hydroxide aqueous solution, and extracted with dichloromethane (30 mL × 3). The extracts were combined, dried over anhydrous sodium sulfate and concentrated under reduced pressure. The residue was separated by rapid preparative chromatograph (dichloromethane/methanol, 10/1, v/v) under medium pressure to obtain Compound 4d (170 mg, 89.5%) as yellow oil. MS: 356.8 [M+H]+.

### STEP D

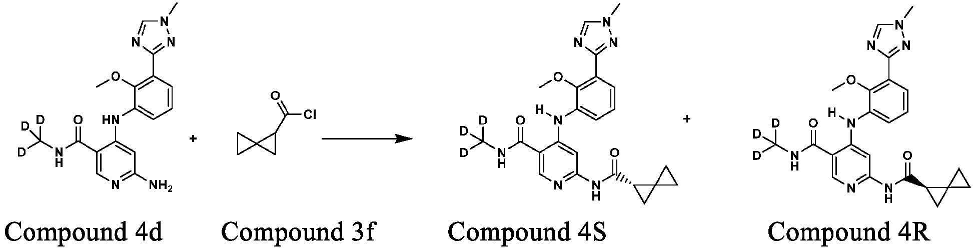

Compound 4d (50 mg, 0.14 mmol) was dissolved in dry tetrahydrofuran (10 mL). Di-trimethylsilylamine lithium (1M in THF, 0.6 mL, 0.6 mmol) was added dropwise at 0°C. The mixture was stirred at 0 °C for 1 hour under nitrogen protection. Then compound 3f (37 mg, 0.28 mmol) was added, and the mixture was stirred at room temperature for 16 hours. The reaction was monitored by LCMS. After the completion of reaction, the reaction was quenched by adding water (20 mL) and extracted with ethyl acetate (30 mL × 2). The extracts were combined, washed with saturated saline, dried over anhydrous sodium sulfate and concentrated under reduced pressure. The residue was separated by rapid preparative chromatograph (dichloromethane/methanol, 10/1, v/v) under medium pressure to obtain 20 mg crude product. The crude product was separated via SFC (Thar prep 80, Column: CHIRALPAK AD-H 250 mm × 20 mm × 5µm, modifier: 40% methanol (NH₄OH 0.2%)/60% CO2, Total flow rate: 40 g/min, Temperature: 40°C) to obtain two isomers: Compound 4S (4.4 mg, Rt: 5.59 min, OR: 228.0, 24.5°C), and Compound 4R (4.2 mg, Rt: 7.07 min, OR: -139.4, 24.5 °C) total yield: 13.6%.

**Compound 4S:** 1H NMR (400 MHz, CDCl3): δ ppm 10.73 (s, 1H), 9.06 (s, 1H), 8.47 (s, 1H), 8.11 (s, 1H), 8.04 (s 1H), 7.77 - 7.75 (m, 1H), 7.55 - 7.52 (m, 1H), 7.28 - 7.26 (m, 1H), 7.06 (s, 1H), 4.01 (s, 3H), 3.81 (s, 3H), 2.04 - 2.01 (m, 1H), 1.58 (t, J = 4.0 Hz, 1H), 1.47 - 1. 44 (m, 1H), 1.06 - 1.02 (m, 1H), 0.97 - 0.90 (m, 3H). LCMS: 451.2 [M+H]+.

**Compound 4R:** 1H NMR (400 MHz, CDC13): δ ppm 10.74 (s, 1H), 9.06 (s, 1H), 8.47 (s, 1H), 8.12 (s, 1H), 8.05 (s 1H), 7.77 - 7.75 (m, 1H), 7.54 - 7.53 (m, 1H), 7.28 - 7.26 (m, 1H), 7.06 (s, 1H), 4.01 (s, 3H), 3.81 (s, 3H), 2.05 - 2.01 (m, 1H), 1.58 (t, J = 4.2 Hz, 1H), 1.47 - 1. 44 (m, 1H), 1.06 - 1.02 (m, 1H), 0.97 - 0.90 (m, 3H). LCMS: 451.2 [M+H]+.

### Example 5:

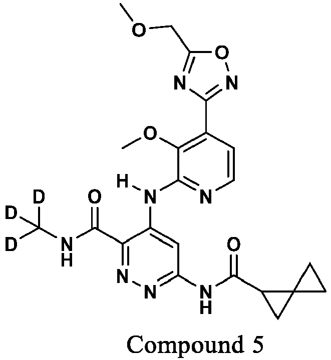

### STEP A

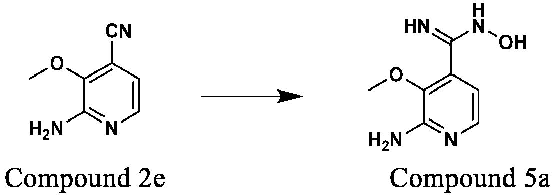

Compound 3a (300 mg, 2 mmol) was added to ethanol (10 mL), followed by the addition of 50% hydroxylamine aqueous solution (528 mg, 8 mmol) and the mixture was stirred at 40°C for 16 hours. The reaction was monitored by LCMS. After the completion of reaction, the reaction was concentrated under reduced pressure. The residue was separated by rapid preparative chromatograph (dichloromethane/methanol, 10/1, v/v) under medium pressure to obtain Compound 5a (310 mg, 85.0%) as yellow solid. 1H NMR (400 MHz, DMSO- d6): δ ppm 9.68 (s, 1H), 7.62 (d, J = 2.4 Hz, 1H), 6.54 (d, J = 2.4 Hz, 1H), 5.95 (s, 2H), 5.76 (s, 2H), 3.66 (s, 3H). LCMS: 182.9 [M+H]+.

### STEP B

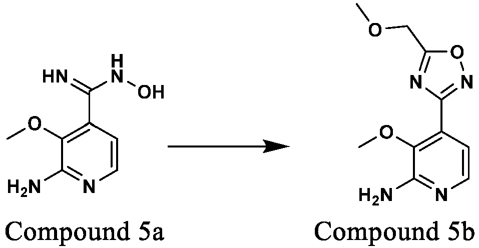

Compound 5a (220 mg, 1.2 mmol) and pyridine (202 mg, 2.55 mmol) were added to dichloromethane (10 mL). Then methoxyacetyl chloride (259 mg, 2.4 mmol) was added dropwise. The mixture was stirred at room temperature for 3 hours under nitrogen protection. Then 1N tetrabutylammonium fluoride in tetrahydrofuran (3 mL, 3 mmol) was added. The mixture was stirred at room temperature for 2 hours. The reaction was monitored by LCMS. After the completion of reaction, the reaction was quenched by adding water (20 mL) and extracted with dichloromethane (30 mL × 2). The extracts were combined, washed with saturated saline, dried over anhydrous sodium sulfate and concentrated under reduced pressure to obtain crude product. The crude product was separated by rapid preparative chromatograph (dichloromethane/methanol, 10/1, v/v) under medium pressure to obtain Compound 5b (140 mg, 49.2%) as yellow solid. LCMS: 237.2 [M+H]+.

### STEP C

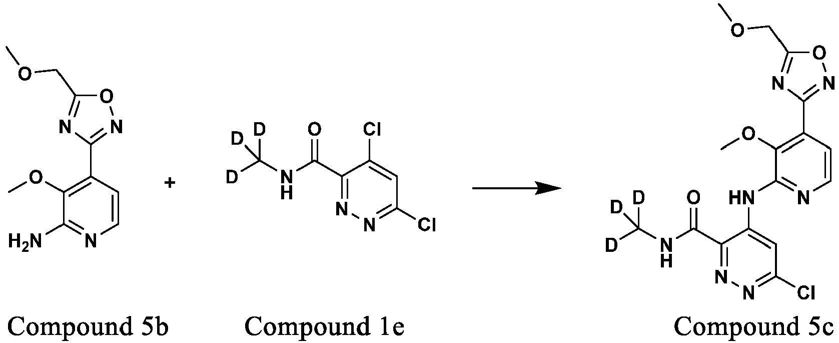

Compound 5b (140 mg, 0.59 mmol) and Compound 1e (160 mg, 0.77 mmol) were dissolved in dry tetrahydrofuran (10 mL). Sodium hydride (60%, 92 mg, 2.4 mmol) was added at 0 °C. The mixture was stirred at room temperature for 4 hours under nitrogen protection. The reaction was monitored by LCMS. After the completion of reaction, the reaction was quenched by adding water (20 mL), concentrated under reduced temperature to obtain crude product. The residue was filtered to obtain Compound 5c (150 mg, 61.0%) as yellow solid. 1H NMR (400 MHz, DMSO- d6): δ ppm 12.67 (s, 1H), 9.50 (s, 1H), 9.19 (s, 1H), 8.33 (d, J = 2.4 Hz, 1H), 7.53 (d, J = 2.4 Hz, 1H), 4.86 (s, 2H), 3.90 (s, 3H), 3.43 (s, 3H). LCMS: 409.1 [M+H]+.

### STEP D

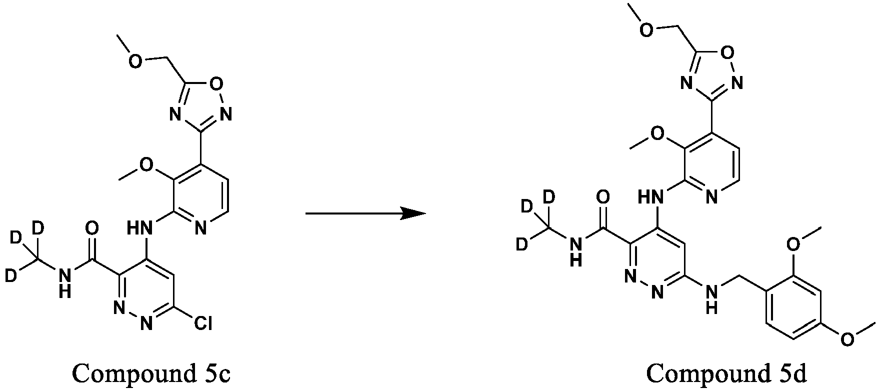

Compound 5c (150 mg, 0.36 mmol) and 2,4-dimethoxybenzylamine (180 mg, 1.08 mmol) were dissolved in dimethyl sulfoxide (10 mL), followed by the addition of potassium fluoride (84 mg, 1.44 mmol). The mixture was stirred at 110°C for 4 hours under nitrogen protection. The reaction was monitored by LCMS. After the completion of reaction, the reaction was diluted by adding water (20 mL) and extracted with ethyl acetate (30 mL × 2). The extracts were combined, washed with saturated saline, dried over anhydrous sodium sulfate and concentrated under reduced pressure to obtain crude product. The crude product was separated by rapid preparative chromatograph (dichloromethane/methanol, 10/1, v/v) under medium pressure to obtain Compound 5d (90 mg, 44.1%) as yellow solid. LCMS: 539.6 [M+H]+.

### STEP E

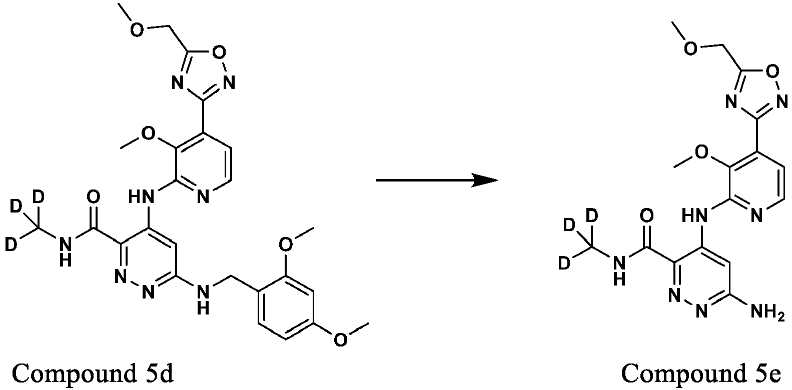

Compound 5d (90 mg, 0.17 mmol) was dissolved in dichloromethane (5 mL). Trifluoroacetic acid (5 mL) was added. The mixture was stirred at room temperature for 4 hours under nitrogen protection. The reaction was monitored by LCMS. After the completion of reaction, the reaction was concentrated under reduced pressure, adjusted to pH=8 using 1N sodium hydroxide solution, and extracted with ethyl acetate (30 mL × 2). The extracts were combined, washed with saturated saline, dried over anhydrous sodium sulfate and concentrated under reduced pressure to obtain crude product. The crude product was separated by rapid preparative chromatograph (dichloromethane/methanol, 10/1, v/v) under medium pressure to obtain Compound 5e (60 mg, 92.7%) as yellow solid. LCMS: 390.1 [M+H]+.

### STEP F

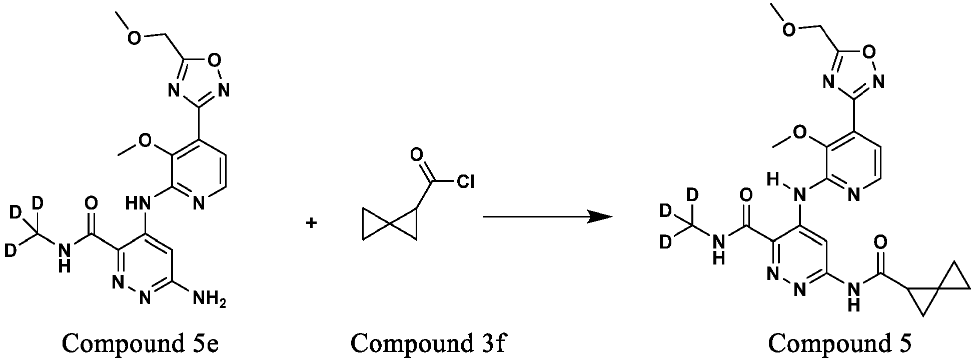

Compound 5e (50 mg, 0.13 mmol) and pyridine (32 mg, 0.4 mmol) were dissolved in 1,2-dichloroethane (10 mL). Then Compound 3f (52 mg, 0.4 mmol) was added dropwise. The mixture was stirred at 80°C for 16 hours under nitrogen protection. The reaction was monitored by LCMS. After the completion of reaction, the reaction was diluted with water (20 mL) and extracted with ethyl acetate (20 mL × 3). The extracts were combined, washed with saturated saline (20 mL), dried over anhydrous sodium sulfate and concentrated under reduced pressure. The residue was separated by rapid preparative chromatograph (dichloromethane/methanol, 10/1-5/1, v/v) under medium pressure to obtain Compound 5 (mixture of isomers, 5 mg, 7.9%). 1H NMR (400 MHz, DMSO-d6): δ ppm 12.56 (s, 1H), 11.20 (s, 1H), 9.90 (s, 1H), 9.29 (s, 1H), 8.30 (d, J = 2.4 Hz, 1H), 7.49 (d, J = 2.4 Hz, 1H), 4.90 (s, 2H), 3.92 (s, 3H), 3.46 (s, 3H), 2.00 - 1.99 (m, 1H), 1.49 - 1.47 (m, 2H), 0.92 - 0.80 (m, 4H). LCMS: 483.6 [M+H]+.

### Example 6:

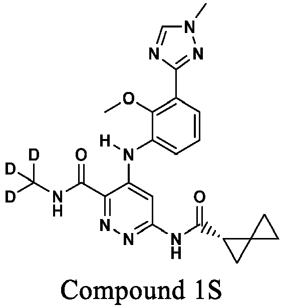

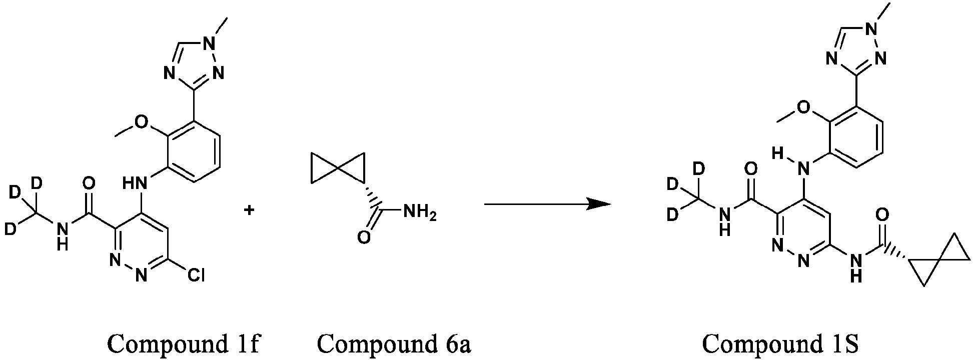

Compound 1f (310mg, 0.82mmol) and Compound 6a (prepared from (S)-spiro[2,2]pentane-1-carboxylic acid, CAS# 249563-58-4) (183mg,1.64mmol) were dissolved in dioxane (30mL). The solution was successively added with XantPhos (142mg, 0.24mmol), cesium carbonate (536mg, 1.64mmol) and XPhosPdG2 (65mg, 0.08mmol). The mixture was stirred at 110°C for 16 hours under nitrogen protection. The reaction was monitored by LCMS. After the completion of reaction, the reaction was diluted with water (20 mL) and extracted with ethyl acetate (20 mL × 3). The extracts were combined, washed with saturated saline (20mL), dried over anhydrous sodium sulfate and concentrated under reduced pressure. The residue was separated by rapid preparative chromatograph (dichloromethane/methanol, 10/1-5/1, v/v) under medium pressure to obtain Compound 1S (105mg).

Compound 1S: 1H NMR (400 MHz, CD3OD): δ ppm 11.10 (s, 1H), 10.96 (s, 1H), 9.10 (s, 1H), 8.53 (s, 1H), 8.14 (s, 1H), 7.64 - 7.61 (m, 1H), 7.50 - 7.48 (m, 1H), 7.28 - 7.24 (m, 1H), 3.91 (s, 3H), 3.69 (s, 3H), 2.49 - 2.38 (m, 1H), 1.36 - 1.29 (m, 2H), 0.87 - 0.69 (m, 4H). MS: 452.2 [M+H]+.

### Example 7:

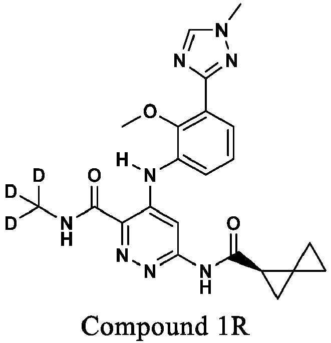

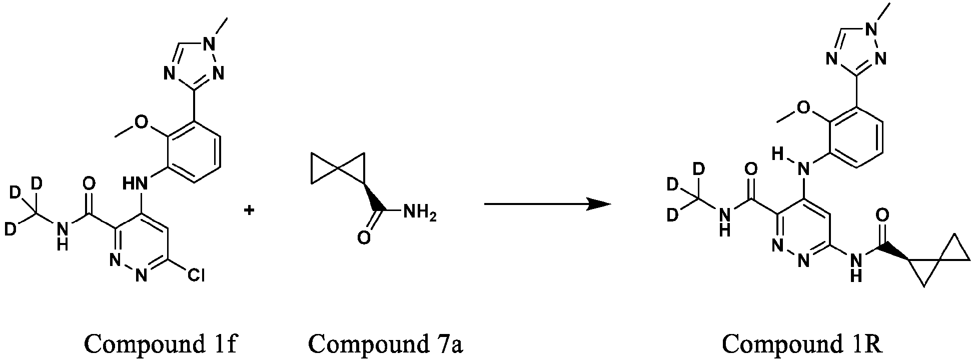

Compound 1f (310mg, 0.82mmol) and Compound 7a (prepared from (R)-spiro[2,2]pentane-1-carboxylic acid, CAS# 249563-53-9) (183mg,1.64mmol) were dissolved in dioxane (30mL). The solution was successively added with XantPhos (142mg, 0.24mmol), cesium carbonate (536mg, 1.64mmol) and XPhosPdG2 (65mg, 0.08mmol). The mixture was stirred at 110°C for 16 hours under nitrogen protection. The reaction was monitored by LCMS. After the completion of reaction, the reaction was diluted with water (20 mL) and extracted with ethyl acetate (20 mL × 3). The extracts were combined, washed with saturated saline (20mL), dried over anhydrous sodium sulfate and concentrated under reduced pressure. The residue was separated by rapid preparative chromatograph (dichloromethane/methanol, 10/1-5/1, v/v) under medium pressure to obtain Compound 1R (100mg). 1H NMR (400 MHz, CD3OD): δ ppm 11.10 (s, 1H), 10.96 (s, 1H), 9.10 (s, 1H), 8.53 (s, 1H), 8.14 (s, 1H), 7.64 - 7.61 (m, 1H), 7.50 - 7.48 (m, 1H), 7.28 - 7.24 (m, 1H), 3.91 (s, 3H), 3.69 (s, 3H), 2.49 - 2.38 (m, 1H), 1.36 - 1.29 (m, 2H), 0.87 - 0.69 (m, 4H). MS: 452.2 [M+H]+.

### Test Example

### Test Example 1: The inhibitory effect of compounds on the expression of pSTAT5 in CD3+cells using FACS detection

### 1. Experimental Materials and Instruments:

### 1.1. Experimental reagents

- DMSO, Sigma, Cat# D2650-100 mL, stored at room temperature.
- Perm buffer III, BD Biosciences, Cat # 558050, stored at 4 °C.
- Lyse/Fix buffer, BD Biosciences, Cat # 558049, stored at room temperature.
- EDTA, Invitrogen, Cat # 15575-038, stored at room temperature.
- PBS, Hyclone, Cat # SH30256.01, stored at 4 °C.
- PE Mouse Anti-Human CD3, BD Biosciences, Cat # 555333, stored at 4 °C.
- Mouse anti-human Phosphorylation-STATS (pY694) (Alexa Fluor^{®} 647 Conjugate), BD Biosciences, Cat# 562076, stored at 4 ° C.
- IFN- α, Biolegend, Cat # 592702

### 1.2 Experimental consumables

- Microplate, 96 Well, PP, v-bottom, Greiner, Cat # GN651201-100EA.
- 5 mL polystyrene round bottom tube, FALCON, Cat # 04318011.
- 96 square well storage plate, Thermo, Cat # AB-0661.
- 96-well plate, Corning, Cat # 3599.

### 1.3 Instruments

- CO2 cell incubator: MCO-15AC Biosciences (Thermo).
- Pipette: 0.2-10 µL, 20-200 µL, 200-1000 µL (Thermo).
- Multichannel pipette: 0.2-10 µL, 5-50 µL, 20-300 µL (Raining).
- Centrifuge: Thermo Centrifuge ST 40R; Thermo LEGEND Micro 21R.
- Water system: Millipore Milli-Q Reference system
- Freezer: Haier ultralow temperature freezer
- Haier 4 degree refrigerator
- Haier -20 degree freezer
- Vortex: EARTH REQUIRED
- Plate Shaker: QI LIN BEI ER; MH-2
- Flow Cytometer: BD FACSVerseTM Flow Cytometer

### 2 Experimental method:

### 2.1 Compound Dilution

1) On the day of experiment, the compound was prepared into a 10mM solution with DMSO, diluted to 1.5mM in DMSO, and then diluted to 8 gradient concentrations in 3-fold dilutions.
2) 5 µL of the diluted compound was transferred into 120ul of DPBS solution containing 0.1% BSA.
3) Positive and negative control groups were set up, with the addition of 0.2% DMSO to the positive and negative control groups.

### 2.2 Experimental procedure

1) Add 0.5 million and 67.5 uL of human PBMC cells to each well of the 96-well cell culture plate.
2) Add 3.5 uL of diluted compound and mix well.
3) Incubate in a 37 °C incubator for 60 minutes.
4) Dilute IFN- alpha in DPBS containing 0.1% BSA to 600ng/mL, and dilute PE-anti-hCD3 antibody in DPBS containing 0.1% BSA to 3 fold. After the above incubation for 60-minute, add diluted PE-anti-hCD3 antibody at 5uL per well and add diluted IFN-α at 4uL per well, i.e., the final concentration of IFN-α per well was 30ng/ml.
5) Incubate in a 37 °C incubator for 30 minutes.
6) Transfer all cells to a 96-well deep well plate and add 1mL of 37 °C preheated lysis/fix buffer.
7) Incubate at 37 °C in dark for 10 minutes.
8) Centrifuge at 600g for 5 minutes and then discard the supernatant. Wash by adding 1mL of PBS for twice and centrifuge.
9) Add 1mL of Perm buffer III to the cell precipitation.
10) Incubate at 4°C in dark for 30 minutes.
11) Centrifuge at 600g for 5 minutes and then discard the supernatant. Wash by adding 1mL of PBS for twice and centrifuge.
12) Dilute APC anti-human pSTAT5 antibody in staining buffer for 200 fold, and add it to cell well at 100 uL per well and mix well.
13) Incubate at room temperature for 40 minutes.
14) Wash twice by adding staining buffer with 1mL for each well, and centrifuge at 600g for 5 minutes.
15) Discard the supernatant, and re-suspend the cell precipitation in 300uL of staining buffer.
16) Sample and analyze in a flow cytometer to obtain the IC50 of the samples to be tested, Table 1.

**Table 1**

| Compound No. | IFN-α/pSTAT5 IC50(nM) |
|---|---|
| 1S | 4.2 |
| 1R | 6.3 |
| 2S | 3.9 |
| 2R | 5.7 |
| 3S | 22.8 |
| 3R | 6.9 |
| 4S | 4.1 |
| 4R | 5.3 |
| 5 | 13 |

### Test Example 2:

Comparison of selectivity towards the JAK family
1. Experimental method
1.1. Pseudokinase (JH2) experimental operation was as follows:
1.1.1. Dissolve the compound with DMSO to a storage concentration of 10mM.
1.1.2. Prepare a compound with a concentration of 200 times the final concentration in a compound dilution plate by diluting it from the highest concentration point for a total of 4 concentration points using a 27 times dilution method, and transfer it to the Echo plate.
1.1.3. Use an Echo instrument to pulse the compound from the Echo plate to a 384 experimental plate, thereby obtain the compound with 11 concentration points of the 3-fold dilution matrix.
1.1.4. Add 5ul 3X TYK2-JH2 or JAK1-JH2 pseudokinase to the 384-well experimental plate.
1.1.5. Add 5ul 3X Tb to the 384-well experimental plate.
1.1.6. Add 5ul 3X Tracer to the 384-well experimental plate.
1.1.7. Centrifuge for 30 seconds and incubate at room temperature for 60 minutes.
1.1.8. Read the signal value using Envision Microplate Reader (PerkinElmer).

1.2. kinase (JH1) experiment operation was as follows:
1.2.1. Dissolve the compound with DMSO to a storage concentration of 10mM.
1.2.2. Prepare a compound with a concentration of 100 times the final concentration in a compound dilution plate by diluting it from the highest concentration point for a total of 4 concentration points using a 27 times dilution method, and transfer it to the Echo plate.
1.2.3. Use an Echo instrument to pulse the compound from the Echo plate to a 384 experimental plate, thereby obtain the compound with 11 concentration points of the 3-fold dilution matrix.
1.2.4. Configure a 2X kinase working solution and add it to a 384-well experimental plate at 5 uL per well. Incubate the compound and the kinase at room temperature for 15 minutes.
1.2.5. Add 5ul 2X substrate (containing ATP) to the 384 well plate.
1.2.6. Incubate at room temperature for 45 minutes.
1.2.7. Add the detection reagent mixture to the 384-well plate, centrifuge for 30 seconds, and incubate at room temperature for 60 minutes.
1.2.8. Read the signal values using Envision Microplate Reader (PerkinElmer).

1.3. Data analysis:
1.3.1. Use XL-Fit software for data analysis to obtain IC50 of the compounds, see Table 2 for results.

**Table 2**

| 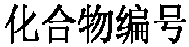 | TYK2 (JH1) (nM) | TYK2(JH2)(nM) | JAK1(JH1)(nM) | JAK1(JH2)(nM) | JAK2(JH1)(nM) | JAK3(JH1)(nM) |
|---|---|---|---|---|---|---|
| 1S | >9950 | 0.13 | >9950 | 0.7 | >9950 | 8671 |
| 1R | >9950 | 0.1 | 1411.9 | 0.9 | >9950 | >9950 |
| 25 | >9950 | 0.13 | 1472.9 | 0.67 | 2188 | 5402.2 |
| 2R | >9950 | 0.2 | 698 | 1.7 | >9950 | 3682 |
| 4S | >9950 | 0.12 | >9950 | 1.66 | >9950 | >9950 |
| 4R | >9950 | 0.1 | 5372 | 3 | >9950 | >9950 |

All documents mentioned in the present invention are cited as references in this application, just as each document is individually cited as a reference. In addition, it should be understood that, after reading the above teaching content of the present invention, those skilled in the art can make various changes or modifications to the present invention, and these equivalent forms also fall within the scope defined by the appended claims of the present application.

## Claims

1. A compound or a pharmaceutically acceptable salt thereof, wherein the compound is of formula A; 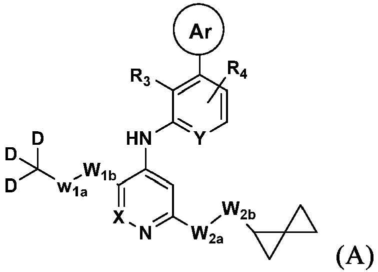 wherein,
Ring Ar is a five-membered heteroaromatic ring; and the five-membered heteroaromatic ring is optionally substituted with 1, 2, 3 or 4 substituents selected from the group consisting of: halogen, substituted or unsubstituted C₁₋₆ alkyl, substituted or unsubstituted C₁₋₆ hydroxyalkyl, substituted or unsubstituted -C₁₋₄ alkylene-O-C₁₋₆ alkyl, substituted or unsubstituted -C₁₋₄ alkylene-S-C₁₋₆ alkyl;
Y is CH or N;
X is CH or N;
R₃ is selected from the group consisting of H, halogen, substituted or unsubstituted C₁₋₆ alkyl, hydroxyl, substituted or unsubstituted C₁₋₆ alkoxy, substituted or unsubstituted C₁₋₆alkylthiol;
R₄ is absent, or means 1 or 2 substituents selected from the group consisting of halogen, substituted or unsubstituted C₁₋₃alkyl;
W₁ₐ and W_{1b} are each independently selected from the group consisting of null (single bond), -O-, -S-, -SO₂-, -CO-, and -NR₅-; and at least one of W₁ₐ and W_{1b} is not null;
W₂ₐ and W_{2b} are each independently selected from the group consisting of null (single bond), -O-, -S-, -SO₂-, -CO-, and -NR₅-; and at least one of W₂ₐ and W_{2b} is not null;
R₅ is each independently selected from the group consisting of: H, substituted or unsubstituted C₁₋₄alkyl;
unless otherwise specified, said substituted means that one or more hydrogen atoms in the group are substituted with substituents selected from the group consisting of halogen, C₁₋₄ alkyl, and C₁₋₄ haloalkyl.

2. A compound or a pharmaceutically acceptable salt thereof, wherein the compound is of formula I; 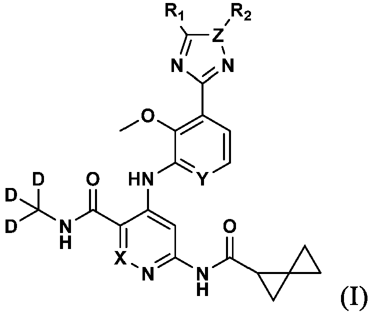 wherein,
R₁ is selected from the group consisting of H, substituted or unsubstituted C₁₋₆ alkyl, substituted or unsubstituted C₁₋₆ hydroxyalkyl, substituted or unsubstituted C₁₋₄ alkylene-O-C₁₋₆ alkyl, substituted or unsubstituted C₁₋₄ alkylene-S-C₁₋₆ alkyl;
Z is selected from the group consisting of O, S, and N;
when Z is O or S, R₂ is absent; when Z is N, R₂ is selected from the group consisting of H, and substituted or unsubstituted C₁₋₆ alkyl;
Y is CH or N;
X is CH or N;
unless otherwise specified, said substituted means that one or more hydrogen atoms in the group are substituted with substituents selected from the group consisting of halogen, C₁₋₄ alkyl, and C₁₋₄ haloalkyl.

3. The compound according to claim 2, wherein when Z is O or S, R₂ is absent; when Z is N, R₂ is substituted or unsubstituted C₁₋₆ alkyl.

4. The compound according to claim 2, wherein R₁ is H; Z is N; and R₂ is selected from the group consisting of: H, substituted or unsubstituted C₁₋₆alkyl.

5. The compound according to claim 2, wherein the compound is of formula Ia or formula Ib

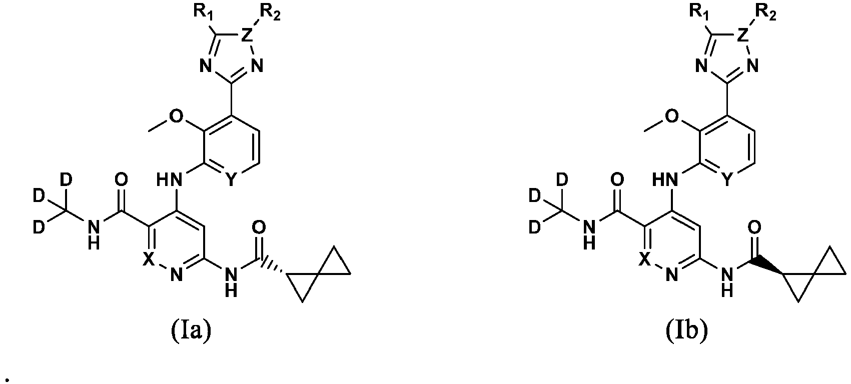

6. The compound according to claim 1 or 2, wherein the compound is a compound selected from Table A:

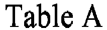

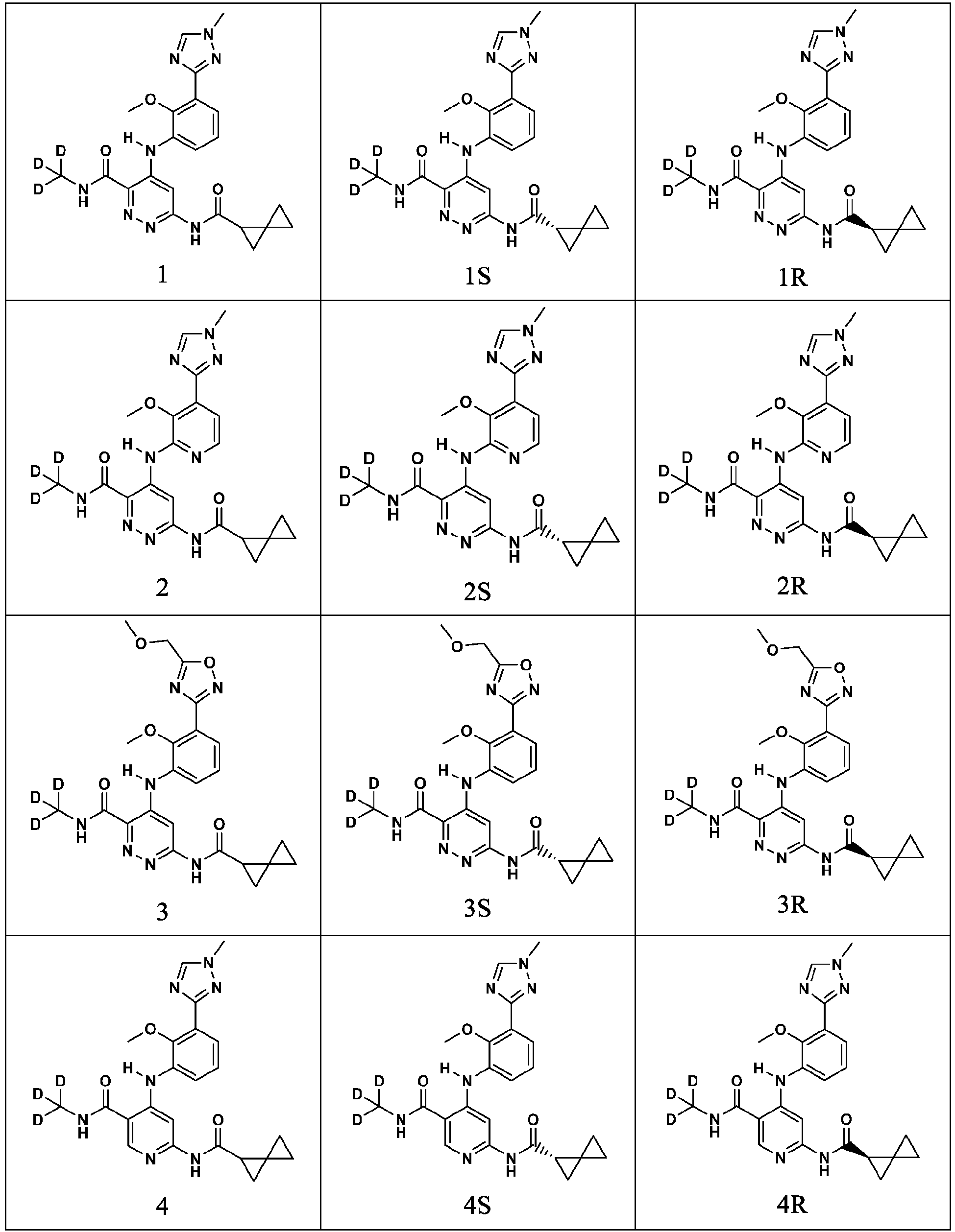

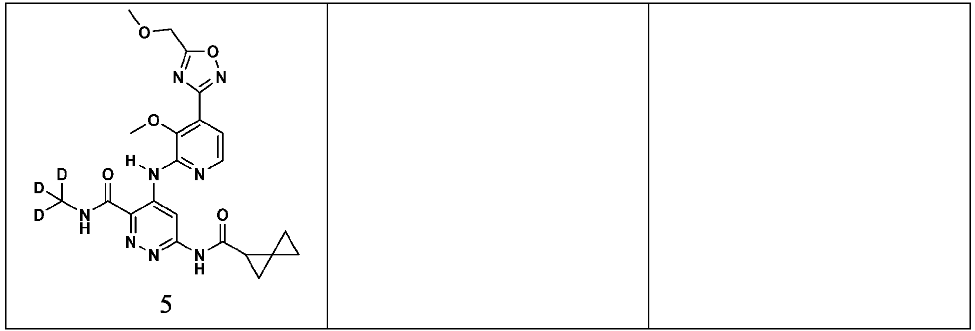

7. A pharmaceutical composition, comprising
(i) the compound according to claim 1 or 2 or the pharmaceutically acceptable salt thereof, and (ii) pharmaceutically acceptable carriers or excipients.

8. A use of the compound according to claim 1 or 2, or the pharmaceutical composition according to claim 6 in the preparation of (i) a drug for treating or preventing a TYK2 and/or JAK1-mediated disease and/or (ii) a TYK2/JAK1 inhibitor.

9. The use according to claim 8, wherein the TYK2 and/or JAK1-mediated disease is selected from the group consisting of inflammation, autoimmune disease, and a combination thereof.

10. The use according to claim 8, wherein the TYK2 and/or JAK1-mediated disease comprises: psoriasis, lupus erythematosus, inflammatory bowel disease, psoriatic arthritis, arthritis, dermatitis, lupus nephritis, neuroinflammation such as multiple sclerosis and senile dementia, ankylosing spondylitis, hidradenitis suppurativa, respiratory disease, diabetes, inflammatory eye disease, hepatitis, cardiovascular disease, systemic sclerosis, organ transplantation, alopecia areata, acne, eczema, vitiligo, sjogren's syndrome, viral inflammation, cancer, or combinations thereof.

11. A preparing method for compound, wherein the compound is as described in claim 2, the preparing method comprises a step of:
reacting an intermediate of formula II with 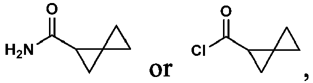 thereby obtaining the compound;
